(19) Europäisches Patentamt
European Patent Office
Office européen des brevets

(11) EP 0 871 653 B1

(12) **EUROPÄISCHE PATENTSCHRIFT**

(45) Veröffentlichungstag und Bekanntmachung des Hinweises auf die Patenterteilung: **26.03.2003 Patentblatt 2003/13**

(21) Anmeldenummer: **96938049.2**

(22) Anmeldetag: **04.11.1996**

(51) Int Cl.[7]: **C07K 5/02**, C07K 5/06, A61K 38/05

(86) Internationale Anmeldenummer: **PCT/EP96/04771**

(87) Internationale Veröffentlichungsnummer: **WO 97/017362 (15.05.1997 Gazette 1997/21)**

(54) **NEUE AMINOSÄUREDERIVATE, VERFAHREN ZU IHRER HERSTELLUNG UND DIESE VERBINDUNGEN ENTHALTENDE PHARMAZEUTISCHE ZUSAMMENSETZUNGEN**

NOVEL AMINO ACID DERIVATIVES, METHODS OF PRODUCING THEM, AND PHARMACEUTICAL COMPOUNDS CONTAINING THESE COMPOUNDS

NOUVEAUX DERIVES D'ACIDE AMINE, PROCEDE DE PRODUCTION CORRESPONDANT ET COMPOSITIONS PHARMACEUTIQUES CONTENANT CES COMPOSES

(84) Benannte Vertragsstaaten:
**AT BE CH DE DK ES FI FR GB GR IE IT LI LU MC NL PT SE**

(30) Priorität: **06.11.1995 DE 19541283**

(43) Veröffentlichungstag der Anmeldung: **21.10.1998 Patentblatt 1998/43**

(73) Patentinhaber: **Boehringer Ingelheim Pharma GmbH & Co.KG**
**55218 Ingelheim am Rhein (DE)**

(72) Erfinder:
- **ESSER, Franz**
  **D-55218 Ingelheim (DE)**
- **SCHNORRENBERG, Gerd**
  **D-55435 Gau-Algesheim (DE)**
- **IGNATOW, Hans-Peter**
  **D-55218 Ingelheim (DE)**
- **GIESLER, Guenther**
  **D-55218 Ingelheim (DE)**
- **JUNG, Birgit**
  **D-55270 Schwabenheim (DE)**
- **SPECK, Georg**
  **D-55218 Ingelheim (DE)**

(74) Vertreter: **Laudien, Dieter, Dr.**
**Boehringer Ingelheim GmbH,**
**Binger Strasse 173**
**55216 Ingelheim (DE)**

(56) Entgegenhaltungen:
**WO-A-94/05693**

- **JOURNAL OF MEDICINAL CHEMISTRY, Bd. 37, Nr. 13, 24.Juni 1994, Seiten 2090-2099, XP000571621 HAGIWARA D ET AL: "STUDIES ON NEUROKININ ANTAGONISTS. 4 SYNTHESIS AND STRUCTURE-ACTIVITY RELATIONSHIPS OF NOVEL DIPEPTIDE SUBSTANCE P ANTAGONISTS: N2 (4R)-4-HYDROXY-1-(1-METHYL-1H-INDOL-3-YL)CARBONYL-L-PROLYL-N-METHYL -N-(PHENYLMETHYL)-3-(2-NAPHTHYL)-L-ALANINAMIDE AND ITS RELATED COMPOUNDS"**

Bemerkungen:
Die Akte enthält technische Angaben, die nach dem Eingang der Anmeldung eingereicht wurden und die nicht in dieser Patentschrift enthalten sind.

EP 0 871 653 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die Erfindung betrifft neue Aminosäurederivate der allgemeinen Formel I

$$R^1\text{-}R^{11}\text{-}A^1\text{-}A^2\text{-}NR^2R^3 \qquad (I)$$

und deren pharmazeutisch annehmbare Salze, Verfahren zu ihrer Herstellung und diese Verbindungen enthaltende pharmazeutische Zusammensetzungen. Die Verbindungen sind wertvolle Neurokinin (Tachykinin)-Antagonisten.

In den europäischen Patentanmeldungen EP 394 989 und EP 443 132 werden Peptide mit Neurokinin antagonistischer Wirkung beschrieben. Die erfindungsgemäßen Verbindungen unterscheiden sich von diesen Peptiden wesentlich in den Gliedern $R^1$, $A^2$, $R^5$ und $-NR^2R^3$.
Den erfindungsgemäßen Verbindungen strukturähnliche Verbindungen mit selektiver NK1-antagonistischer Wirkung werden in WO 94/05 693 und in J. Med. Chem., Vol. 37(13), pp. 2090-99 (1994) beschrieben.

Die in dieser Beschreibung und den Ansprüchen für die Aminosäuren verwendeten Abkürzungen entsprechen dem üblichen Dreibuchstabencode wie er z.B. in Europ. J. Biochem., 138, 9 (1984) beschrieben ist. Die übrigen Abkürzungen werden nachfolgend erklärt:

| | |
|---|---|
| Boc = | t-Butoxycarbonyl |
| Bzl = | Benzyl |
| CDI = | Carbonyldiimidazol |
| Cha = | 3-Cyclohexylalanin |
| DCCI = | Dicyclohexylcarbodiimid |
| DCH = | Dicyclohexylharnstoff |
| HOBt = | 1-Hydroxybenztriazol |
| Hpa = | Homophenylalanin |
| Hyp = | (2S,4R)-Hydroxyprolin |
| Pal = | 3-(1-Pyrrolyl)alanin |
| THF = | Tetrahydrofuran |
| TFA = | Trifluoressigsäure |
| Z = | Benzyloxycarbonyl |
| Me = | Methyl |
| Ac = | Acetyl |
| Et = | Ethyl |
| DMF = | Dimethylformamid |
| DPPA = | Diphenylphosphorylazid |
| PPA = | Polyphosphorsäure |
| RT = | Raumtemperatur |
| Mtr = | 4-Methoxy-2,3,6-trimethylbenzolsulfonyl |
| Trp(for) = | formylgeschütztes Triptophan |
| Met(0) = | Methionin, worin S zum Sulfoxid oxidiert ist. |
| Bum = | N(π)-tert. butoxymethyl |

Der Ausdruck Aminosäure umfaßt (falls im folgenden Text nicht ausdrücklich etwas anderes angegeben ist) natürliche und unnatürliche Aminosäuren, sowohl der D-als auch der L-Form, insbesondere α-Aminosäuren, sowie deren Isomere.

Wenn eine Aminosäure ohne Präfix angegeben ist, steht diese Angabe für die L-Form der Aminosäure.
Die D-Form wird ausdrücklich angegeben.

Für die Darstellung der Formeln wird eine vereinfachte Darstellung verwendet. Dabei werden in der Darstellung von Verbindungen alle $CH_3$-Substituenten jeweils durch einen Bindungsstrich dargestellt, so steht zum Beispiel

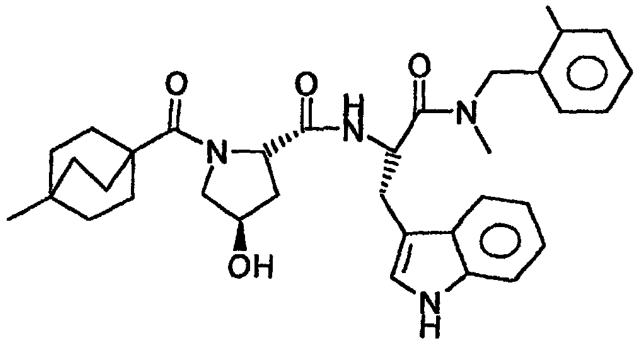

für

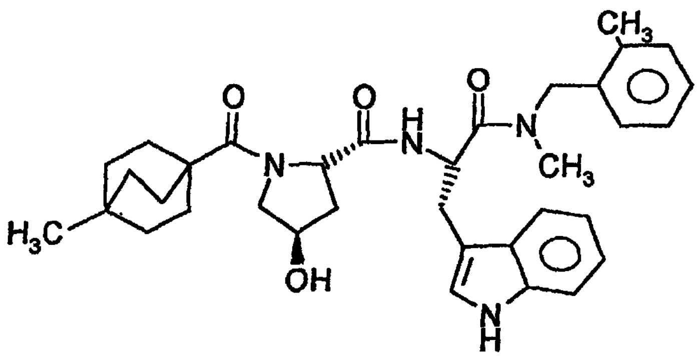

Die Erfindung betrifft neue Aminosäurederivate der allgemeinen Formel I

$$R^1\text{-}R^{11}\text{-}A^1\text{-}A^2\text{-}NR^2R^3 \qquad (I)$$

und deren pharmazeutisch annehmbare Salze, worin

$R^1$ eine der Gruppen

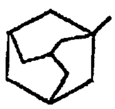
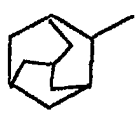
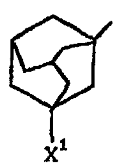
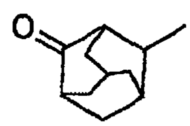
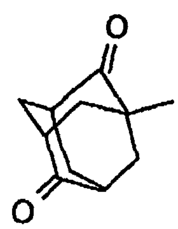

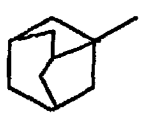
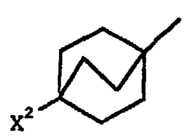
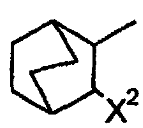

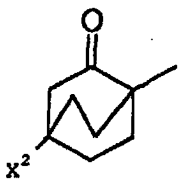
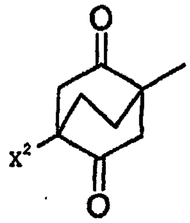
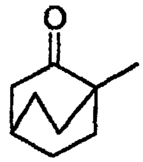
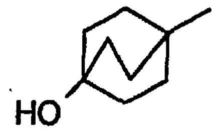

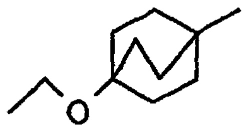
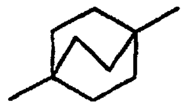

oder

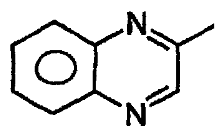

ist,
worin

X1 Halogen, COOH, $C(O)NH_2$, C(O)Oalkyl, C(O)NHalkyl, $C(O)N(alkyl)_2$, [worin Alkyl für Methyl, Ethyl, Propyl, Butyl oder Pentyl steht],

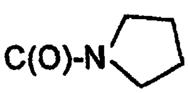, 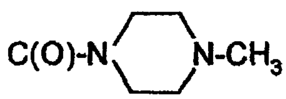,

$CH_2NH_2$, $CH_2NH$ alkyl, $CH_2N(alkyl)_2$ [worin Alkyl für Methyl, Ethyl, Propyl, Butyl oder Pentyl steht],

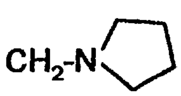, 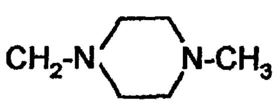,

CN, $NH_2$ oder NH(Sch) [worin Sch für Methyloxycarbonyl, Ethyloxycarbonyl oder Phenyl-($C_1$ oder 2 alkyl)oxycarbonyl steht, wobei das Phenyl unsubstituiert ist oder durch Halogen, ($C_1$-$C_5$)Alkyl oder ($C_1$-$C_5$)Alkoxy substituiert ist] und

X2 Halogen, Alkyl, OH, O-alkyl, C(O)Oalkyl, COOH, $C(O)NH_2$, C(O)Oalkyl, C(O)NHalkyl, $C(O)N(alkyl)_2$, [worin Alkyl für Methyl, Ethyl, Propyl, Butyl oder Pentyl steht], CN, $NH_2$ oder NH(Sch) [worin Sch für Methyloxycarbonyl, Ethyloxycarbonyl oder Phenyl - ($C_{1\ oder\ 2}$ alkyl)oxycarbonyl steht, wobei das Phenyl unsubstituiert ist oder durch Halogen, ($C_1$-$C_5$)alkyl oder ($C_1$-$C_5$)alkoxy substituiert ist],

bedeutet.

$A^1$ D- oder L-Serin (Ser), D- oder L-Threonin (Thr), D- oder L-alloThreonin, D- oder L- Prolin (Pro), D-oder L-Didehydroprolin (Δ Pro), D- oder L-Hydroxyprolin (Pro(OH)), D- oder L-Thiazolidin-4-carbonsäure, D- oder L- Aminoprolin ($Pro(NH_2)$) , D- oder L- Pyroglutaminsäure (pGlu), D- oder L-Hydroxypiperidincarbonsäure, wobei enthaltene Hydroxy- und Aminogruppen durch übliche Schutzgruppen (z.B. Acyl, Carbamoyl oder Aralkyl geschützt sein können;

$A^2$ eine lipophile α-Aminosäure ist, die eine Phenyl-, 1-, 2- oder 3-fach substituierte Phenyl-, Heteroaryl-, oder eine Naphthylgruppe enthält, und diese Ringgruppe durch $-CH_2-$ oder $-CH_2-CH_2-$ vom Backbone der Aminosäure getrennt ist, wobei die Substituenten der Phenylgruppe unabhängig voneinander Halogen, Trihalogenomethyl, Alkoxy oder Alkyl sind;

$R^2$ und $R^3$ unabhängig voneinander Alkyl, Arylalkyl oder Heteroarylalkyl bedeuten (worin Aryl für Phenyl, 1, 2 oder 3-fach substituiertes Phenyl oder Naphthyl steht; die Substituenten der Phenylgruppe unabhängig voneinander Halogen, Trihalogenomethyl, Alkoxy, Alkyl, Alkylthio, Hydroxy, Trifluormethoxy, Dialkylamino oder Cyano sind oder 2 benachbarte Positionen der Phenylgruppe durch $-O-(CH_2)_{1\ oder\ 2}-O-$ verbunden sind; Heteroaryl für Indolyl, Pyridyl, Pyrrolyl, Imidazolyl oder Thienyl steht; und die Alkyl- bzw. Alkoxygruppe 1 bis 3 Kohlenstoffatome enthält) oder die Gruppe

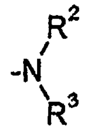

einen Ring der allgemeinen Formel

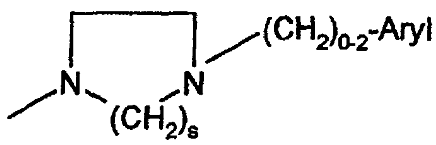

bedeutet, worin s 2 oder 3 ist,
(worin Aryl für Phenyl, 1, 2 oder 3-fach substituiertes Phenyl oder Naphthyl steht; die Substituenten der Phenylgruppe unabhängig voneinander Halogen, Trihalogenomethyl, Alkoxy, Alkyl, Cyano, Hydroxy, Nitro, $-CO_2CH_3$, $-CO_2C_2H_5$ oder Alkylthio sind oder 2 benachbarte Positionen der Phenylgruppe durch $-O-(CH_2)_{1-2}-O-$ verbunden sind und Alkyl 1 bis 3 C-Atome enthält).

Die erfindungsgemäßen Verbindungen sind wertvolle Neurokinin (Tachykinin)-Antagonisten, die sowohl Substanz P-Antagonismus, als auch Neurokinin A- bzw. Neurokinin-B-antagonistische Eigenschaften besitzen. Sie sind nützlich zur Behandlung von und zur Vorbeugung gegenüber Neurokinin-vermittelten Krankheiten.

Verbindungen der allgemeinen Formel I können Säuregruppen besitzen, hauptsächlich Carboxylgruppen, oder phenolische Hydroxygruppen, und/oder basische Gruppen wie z.B. Guanidino- oder Aminofunktionen. Verbindungen der allgemeinen Formel I können deshalb entweder als innere Salze, als Salze mit pharmazeutisch verwendbaren anorganischen Säuren wie Salzsäure, Schwefelsäure, Phosphorsäure, -Sulfonsäure oder organischen Säuren (wie beispielsweise Maleinsäure, Fumarsäure, Zitronensäure, Weinsäure oder Essigsäure) oder als Salze mit pharmazeutisch verwendbaren Basen wie Alkali- oder Erdalkalimetallhydroxiden oder Carbonaten, Zink- oder Ammoniumhydroxiden oder organischen Aminen wie z.B. Diethylamin, Triethylamin, Triethanolamin u.a. vorliegen.

Die Chiralitätszentren in den neuen Aminosäurederivaten können jeweils R-, S- oder R,S-Konfiguration besitzen.

Der in der Definition von $A^2$ enthaltene Ausdruck "Heteroarylgruppe" steht für ein mono-, bi- oder tricyclisches aromatisches Ringsystem, das 1 oder 2 Heteroatome enthält, nämlich ein oder zwei Stickstoffatome oder ein Stickstoffatom und ein Schwefelatom. Die Gruppe kann gewünschtenfalls 1 oder 2 Substituenten ($C_{1-3}$Alkyl) oder eine Oxogruppe oder eine 1-3 C-Atome enthaltende Alkoxygruppe enthalten.

Beispiele geeigneter Heteroarylgruppen sind

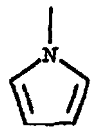 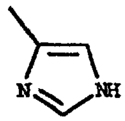 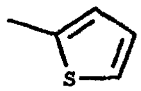 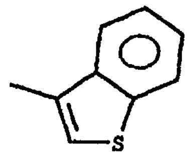 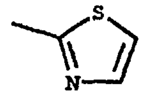

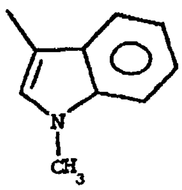 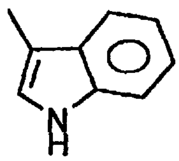 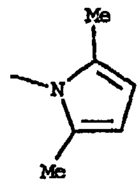 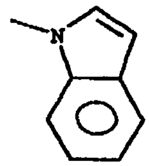

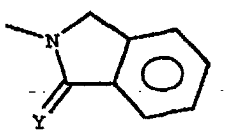 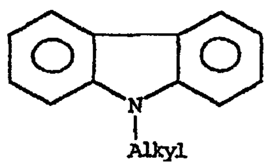 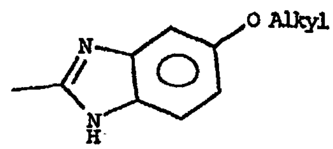

Y = $H_2$ oder O

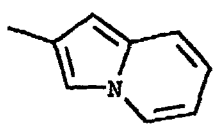 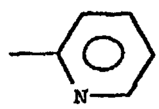

Es ist zu beachten, daß die oben angegebenen Heteroarylgruppen auch in anderen Positionen als die angegebenen an die $-CH_2-CH_2-$Gruppe gebunden sein können.
Die $-CH_2-CH_2-$Gruppe ist an das α-Kohlenstoffatom der Aminosäure ($A^2$) gebunden.
Von den erfindungsgemäßen Verbindungen der Formel I sind solche bevorzugt, worin
$A^1$ Prolin, 4-Hydroxyprolin oder Thiazolidin-4-carbonsäure ist.
Von den oben definierten Verbindungen sind solche bevorzugt, worin $A^1$ 4-Hydroxyprolin mit 2-S-Konfiguration ist.
Ferner sind solche Verbindungen hervorzuheben, worin $A^1$

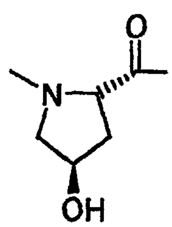

bedeutet.

Weiterhin bevorzugt sind Verbindungen, worin $A^2$ Naphthyl, Indolyl oder N-Methylindolyl ist.

Insbesondere bevorzugt sind Verbindungen, worin $A^2$

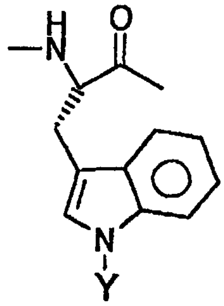

oder

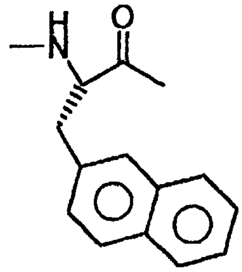

ist, wobei

und Y H oder $CH_3$ bedeutet.

Darüber hinaus bevorzugt sind Verbindungen, wom $R^2$ H oder $CH_3$ ist und $R^3$ Benzyl, wobei die darin enthaltene Phenylgruppe durch Methyl, Chlor oder Brom substituiert ist.

Weiterhin bevorzugt sind Verbindungen, worin $R^3$

2-Methylbenzyl oder 2-Brombenzyl ist.

Weiterhin bevorzugt sind Verbindungen, worin die Gruppe

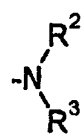

einen Ring

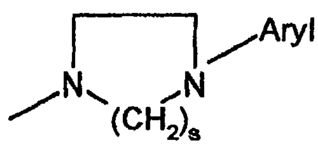

bedeutet, worin s 2 ist.

Besonders hervorzuheben sind folgende Verbindungen:

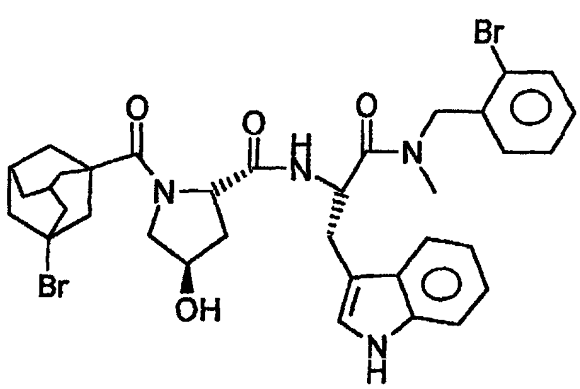

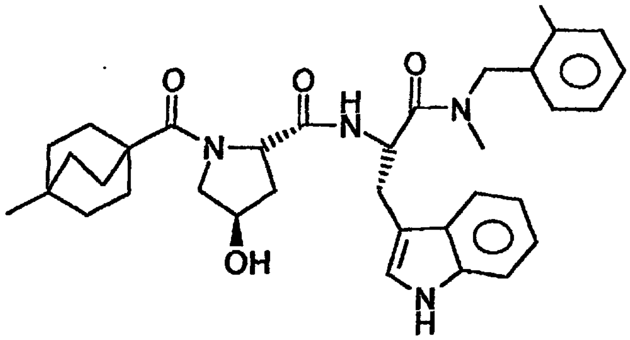

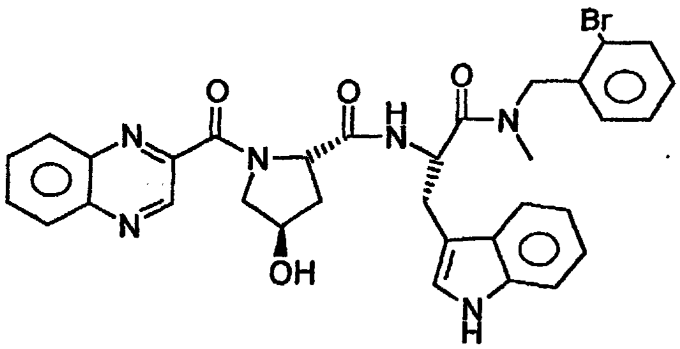

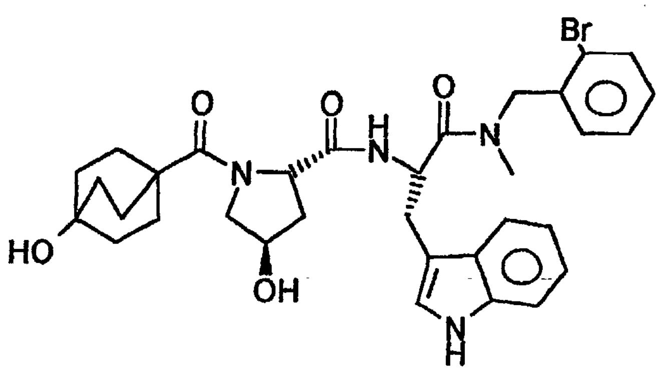

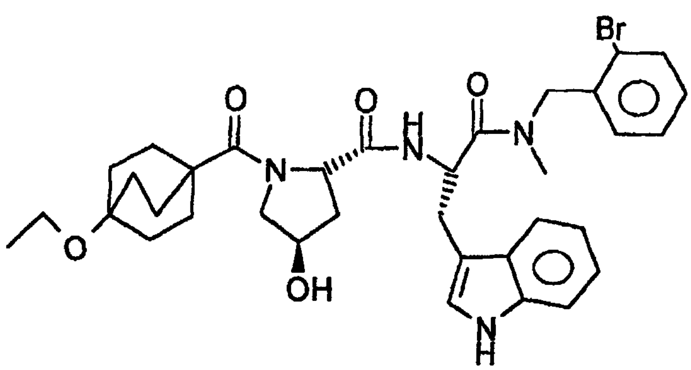

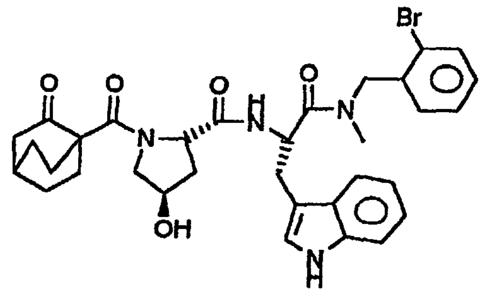

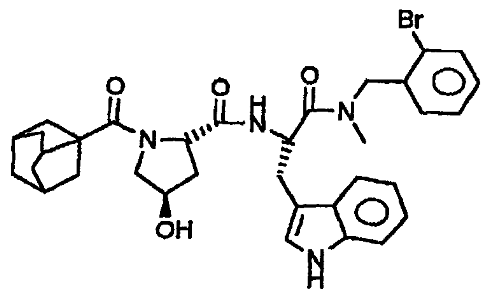

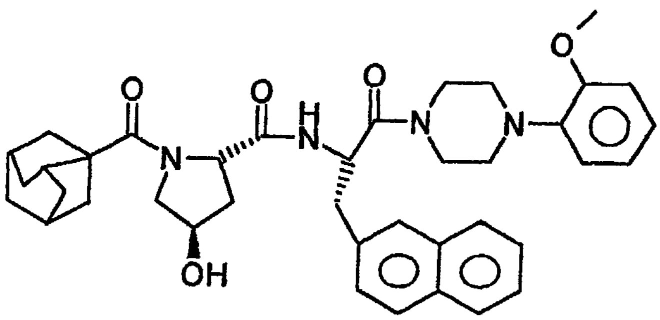

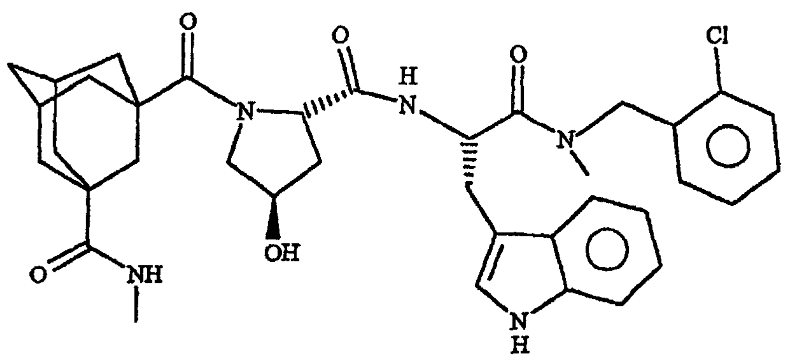

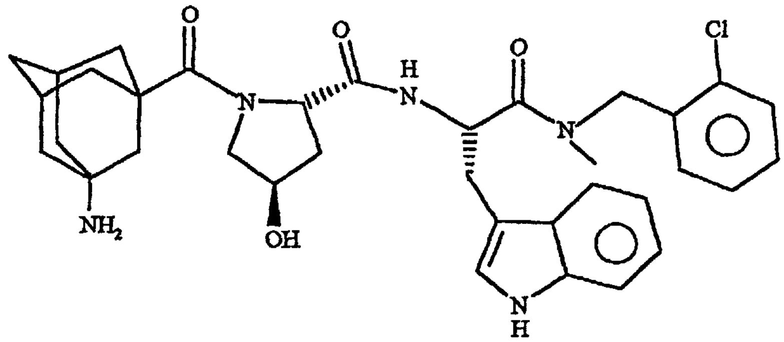

und

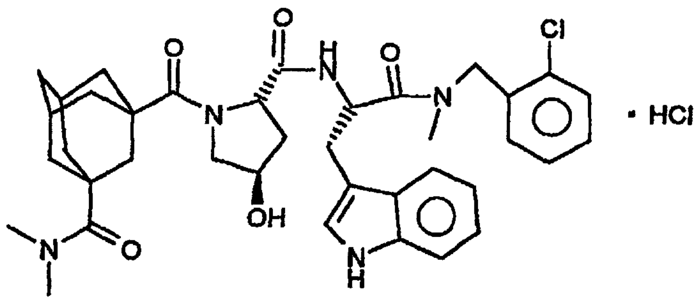

oder deren pharmazeutisch annehmbare Salze.

Die angegebenen Aminosäuren liegen vorzugsweise in S-Konfiguration vor.

Untersuchungsergebnisse für erfindungsgemäße Verbindungen: Die Rezeptoraffinität zum $NK_1$-Rezeptor (Substanz P-Rezeptor) wurde an humanen Lymphoblastoma-Zellen (IM-9) mit klonierten $NK_1$-Rezeptoren bestimmt, wobei die Verdrängung von $^{125}$J-markierter Substanz P gemessen wird. Der $NK_2$-Bindungs-Test wird an transfizierten A20-Zellen durchgeführt, die den humanen $NK_2$-Rezeptor exprimieren. Die Verdrängung von $^{125}$J-BN-Neusolinin A wird bestimmt.

Die so erhaltenen $Ki_{50}$-Werte betragen:

| Verbindung | $NK_1$ [nM] | $NK_2$ [nM] |
|---|---|---|
| **3** | **1** | **55** |
| **5** | **1,3** | **105** |
| **12** | **13** | **52** |
| **13** | **3** | **177** |
| **14** | **5** | **100** |
| **21** | **4,1** | **137** |
| **22** | **6,2** | **45** |
| **29** | **1,1** | **38** |
| **39** | **0,28** | **68** |
| **44** | **0,41** | **122** |
| **50** | **0,4** | **77** |

Zusammenstellung der Beispiele:

1.)

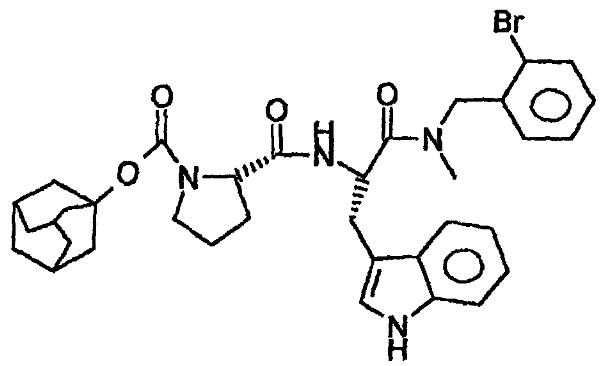

Fp.: 120-128°C; $[\alpha]^{20}_{D}$ = -16,4° (DMSO)

2.)

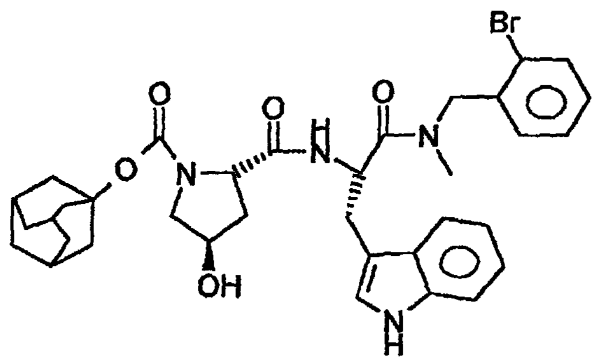

Fp.: 98-128°C; $[\alpha]^{20}_{D}$ = -19,0° (DMSO)

3.)

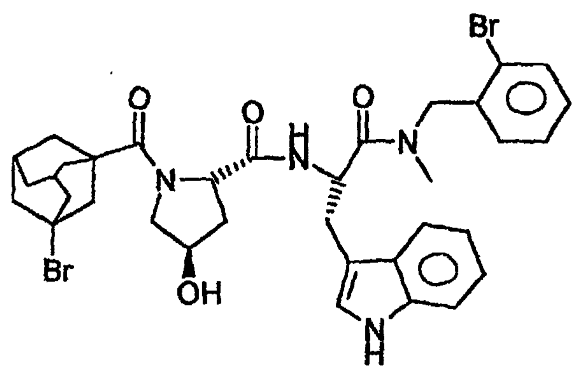

Fp.: 187-190°C; $[\alpha]^{20}_{D}$ = -8,4° (DMSO)

4.)

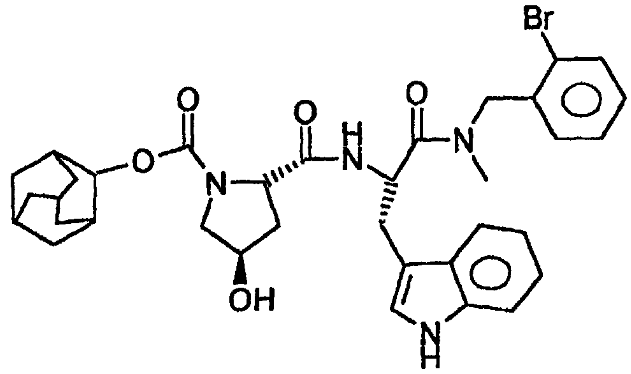

Fp.: 124-130°C; $[\alpha]_D^{20}$ = -19,1° (DMSO)

5.)

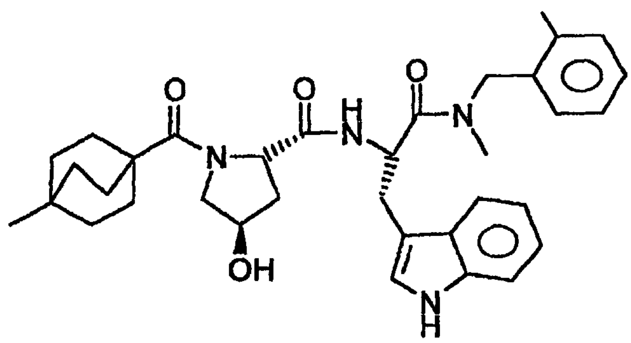

Fp.: 187-189°C; $[\alpha]_D^{20}$ = -11,4° (DMSO)

6.)

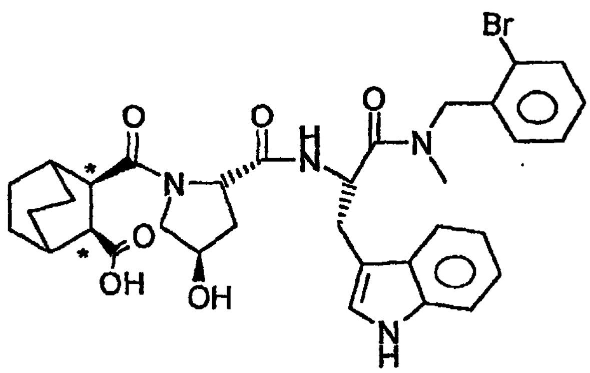

* = S,R od. R,S

Fp.: 189-195°C; $[\alpha]_D^{20}$ = -41,2° (DMSO)

7.)

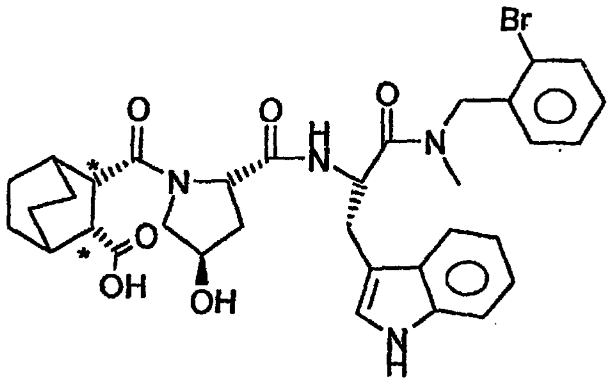

* = R,S od. S,R

Fp.: 168-174°C; $[\alpha]^{20}_{D}$ = -38° (DMSO)

8.)

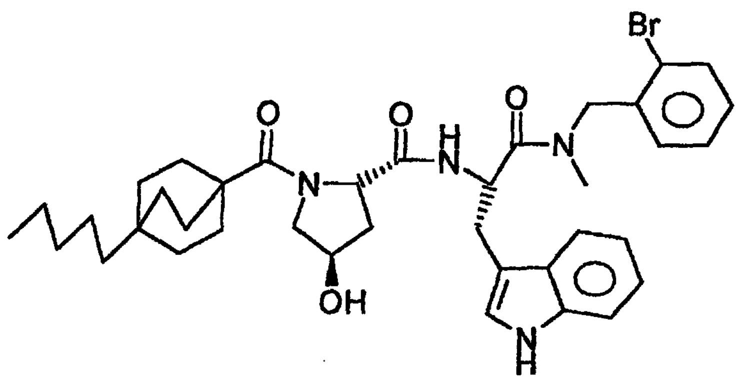

Fp.: 201-204°C; $[\alpha]^{20}_{D}$ = -11,0° (DMSO)

9.)

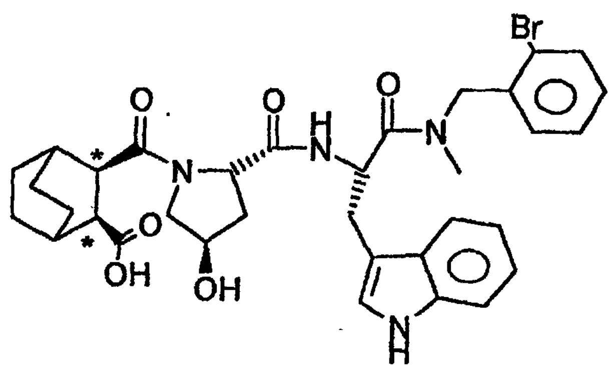

* = S,R od. R,S

Fp.: 184-188°C; $[\alpha]^{20}_{D}$ = -7,6° (DMSO)

10.)

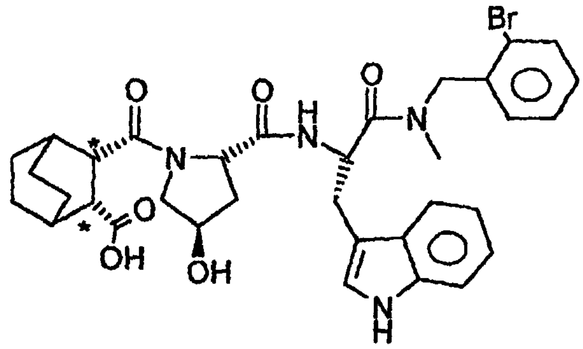

* = R,S od.
S, R

Fp.: 160-165°C; $[\alpha]^{20}_{D}$ = -37,8° (DMSO)

11.)

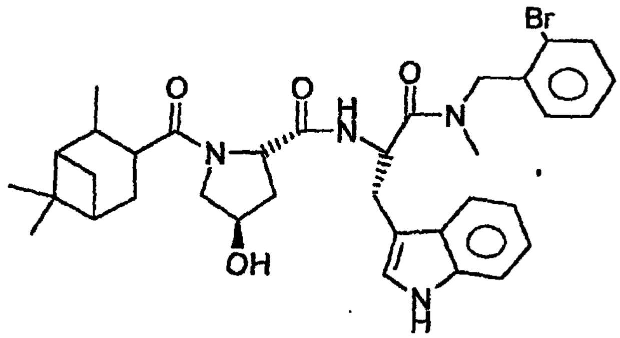

$R^1$-C(O)-: (+) - Pinancarbonyl

Fp.: 120-123°C; $[\alpha]^{20}_{D}$ = -10,3° (DMSO)

12.)

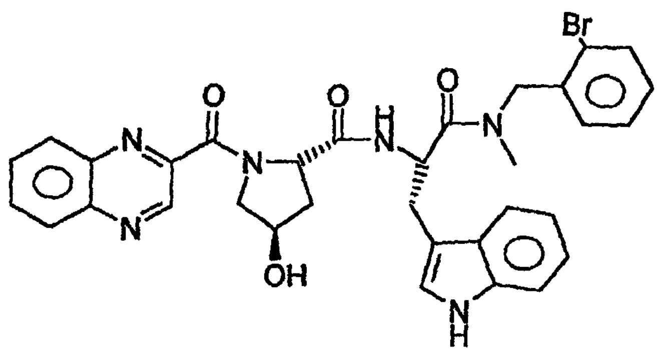

Fp.: 136-140°C; $[\alpha]^{20}_{D}$ = -140,8° (DMSO)

13.)

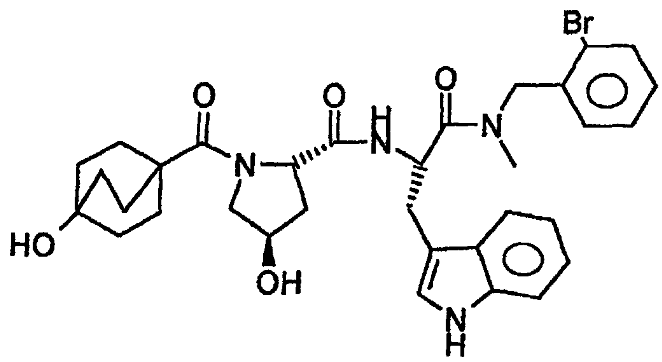

Fp.: 114-118°C; $[\alpha]_D^{20}$ = -12,0° (DMSO)

14.)

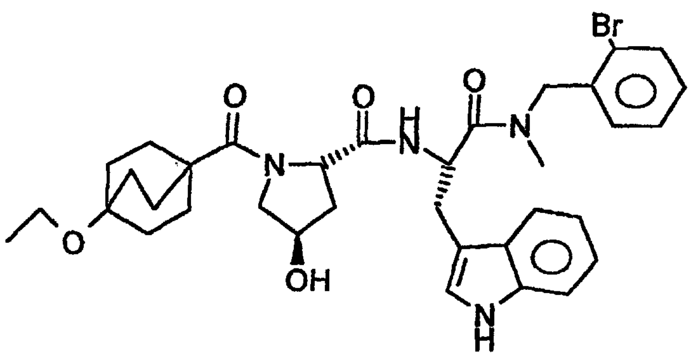

Fp.: 119-126°C; $[\alpha]_D^{20}$ = -11,2° (DMSO)

15.)

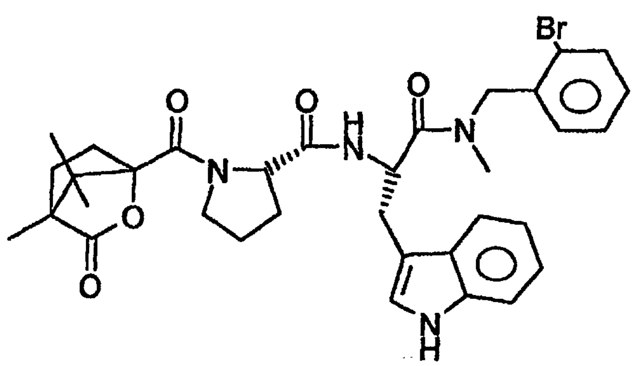

Fp.: 115°C; $[\alpha]_D^{20}$ = -20,6° (DMSO)

16.)

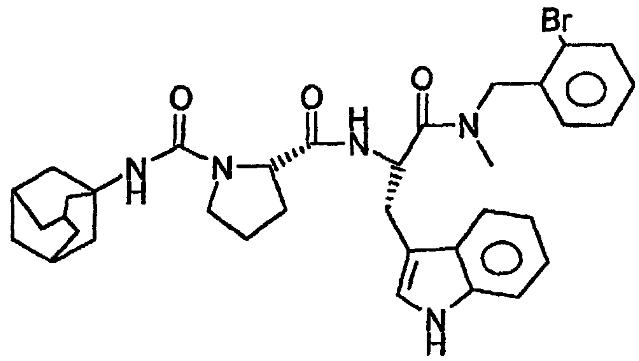

Fp.: 124 - 127°C; $[\alpha]^{20}_{D}$ = -20,4° (DMSO)

17.)

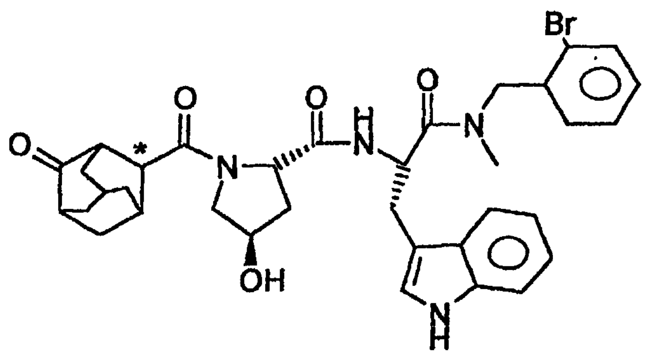

* = R/S

Fp.: 128 - 134°C; $[\alpha]^{20}_{D}$ = -32,8° (DMSO)

18.)

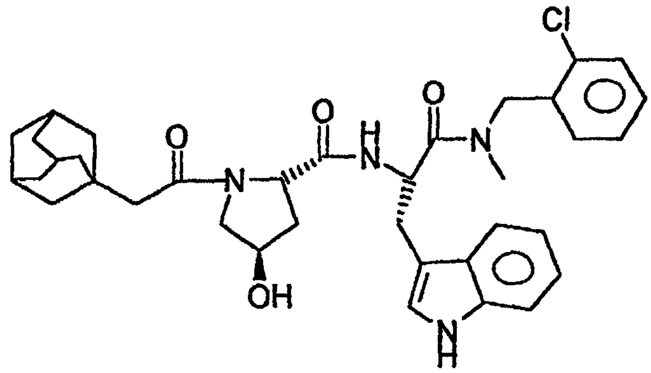

Fp.: 120 - 127°C; $[\alpha]^{20}_{D}$ = -25° (DMSO)

19.)

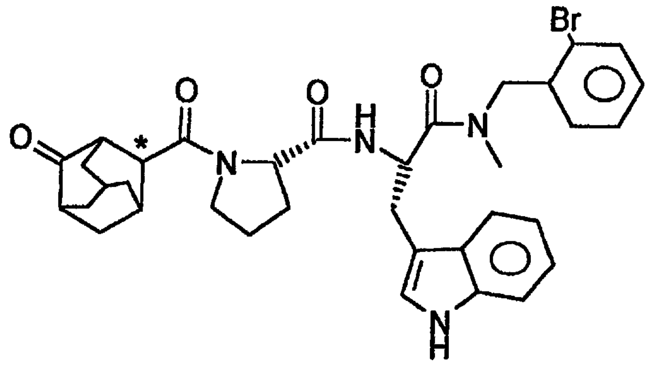

* = R/S

Fp.: 128 - 134°C; $[\alpha]_D^{20}$ = -35,6° (DMSO)

20.)

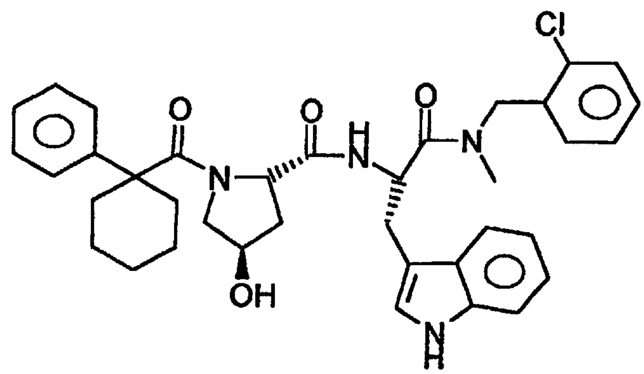

Fp.: 130 - 136°C; $[\alpha]_D^{20}$ = -26,2° (DMSO)

21.)

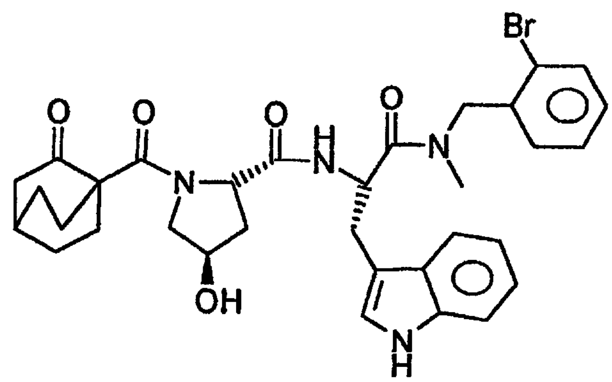

Fp.: 143°C; $[\alpha]_D^{20}$ = -4,8° (DMSO)

22.)

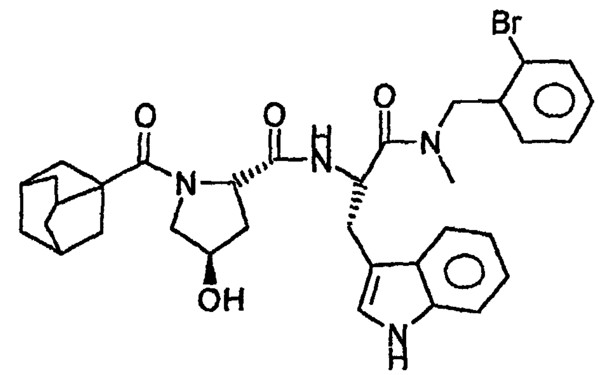

Fp.: 160 - 165°C; $[\alpha]^{20}_{D}$ = -7,0° (DMSO)

23.)

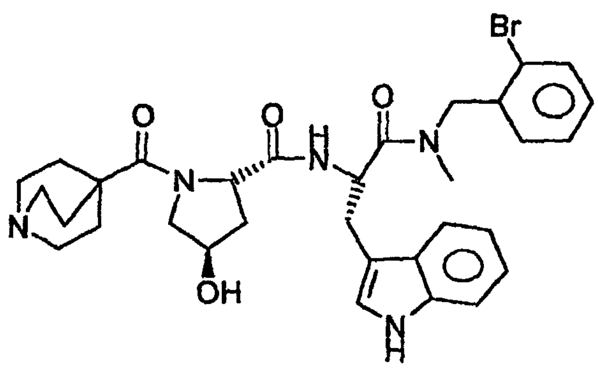

· HCl

Fp.: 196 - 201°C; $[\alpha]^{20}_{D}$ = -11,8° (DMSO)

24.)

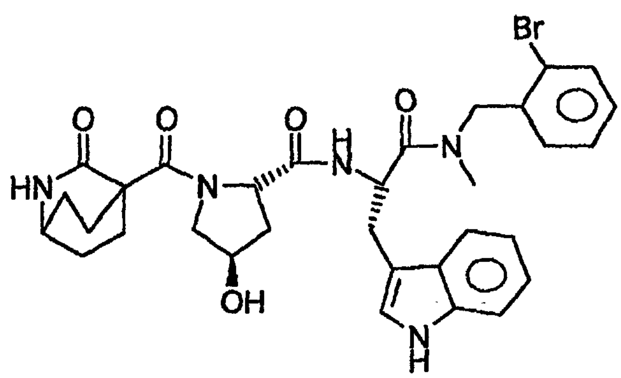

Fp.: 158 - 162°C; $[\alpha]^{20}_{D}$ = -10,8 (DMSO)

25.)

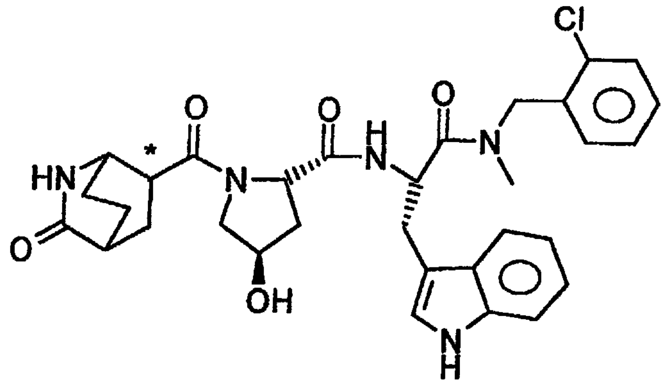

* = R/S

26.)

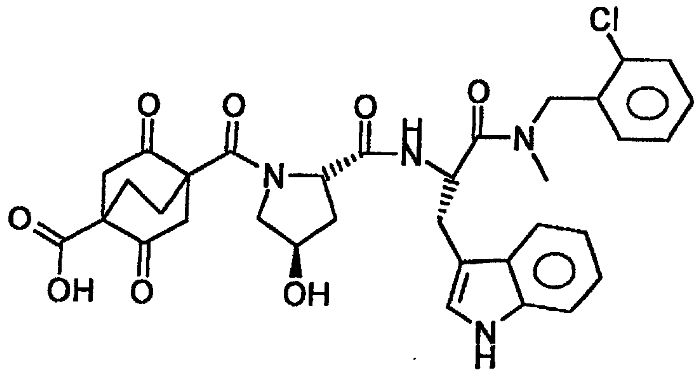

27.)

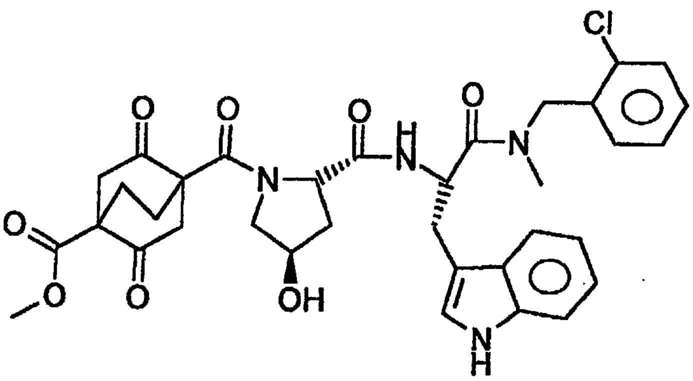

28.)

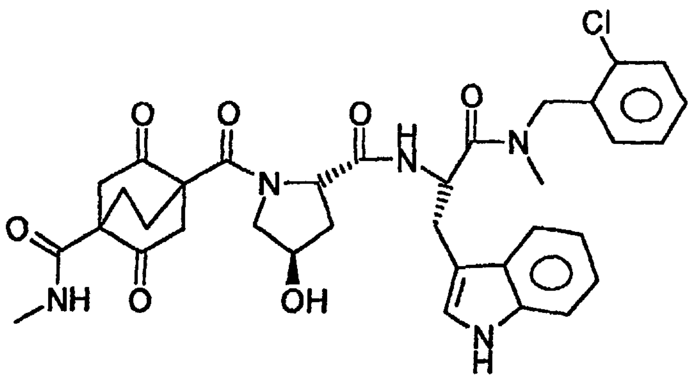

29.)

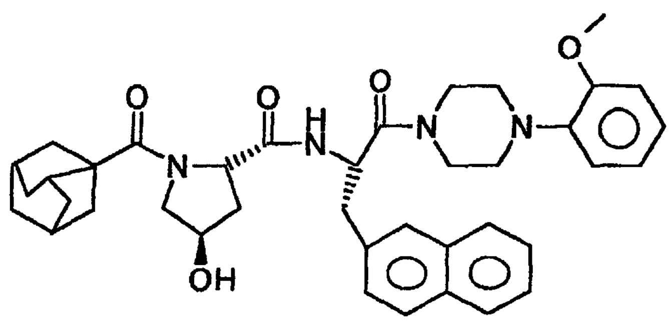

Fp.: 171-178 °C; $[\alpha]_D^{20}$ = - 37, 4 ° (DMSO).

30.)

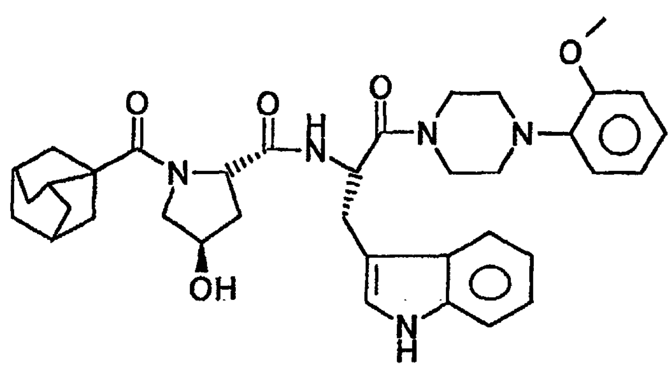

31.)

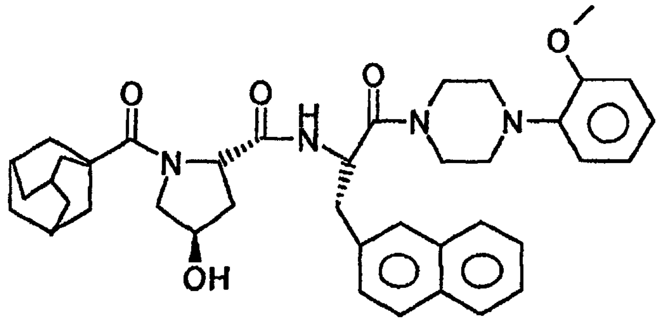

Fp.: 185°C; $[\alpha]_D^{20}$ = -37,2° (DMSO)

32.)

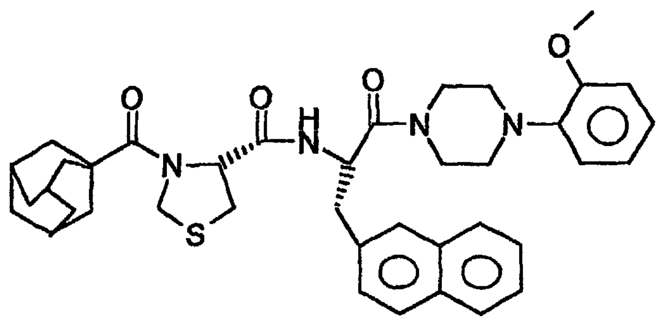

33.)

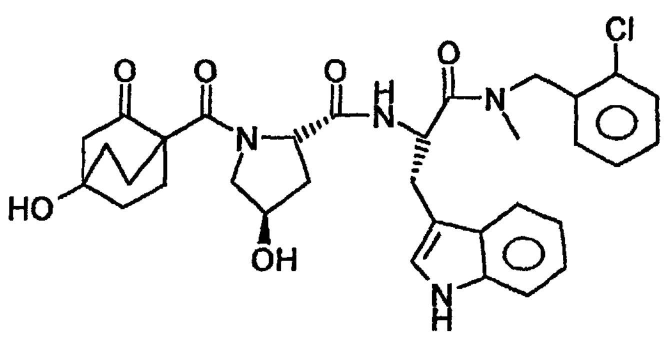

34.)

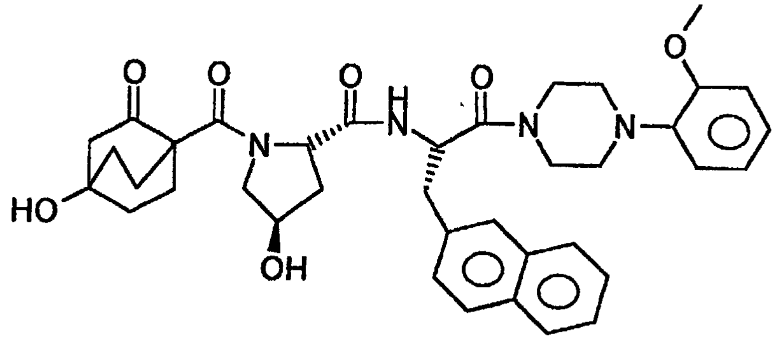

35.)

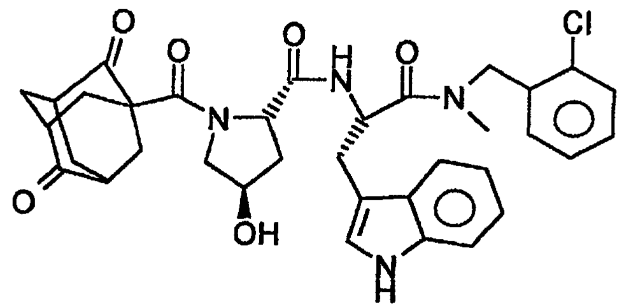

36.)

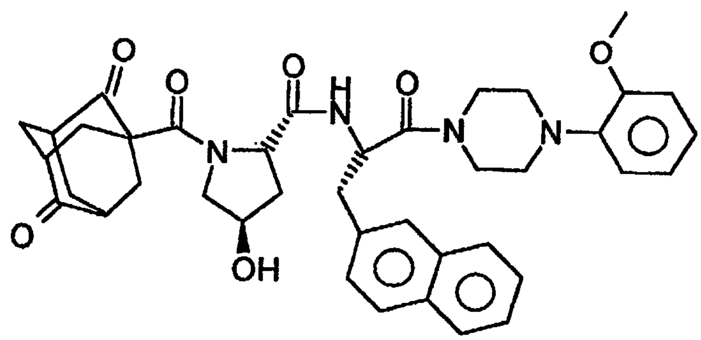

37.)

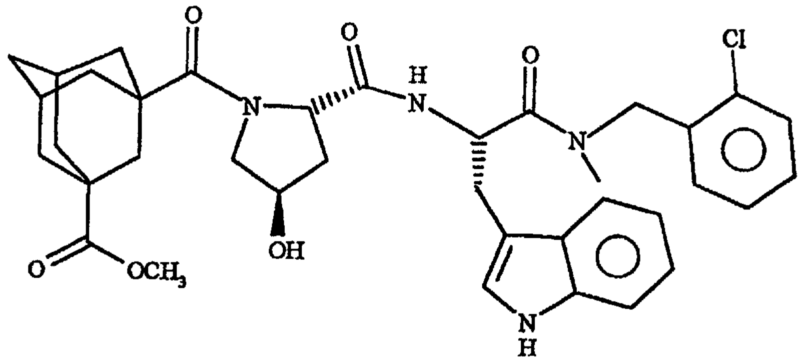

Fp.: 198-200 °C.
$[\alpha]_D^{20}$ = - 5,8 ° (DMSO).

38.)

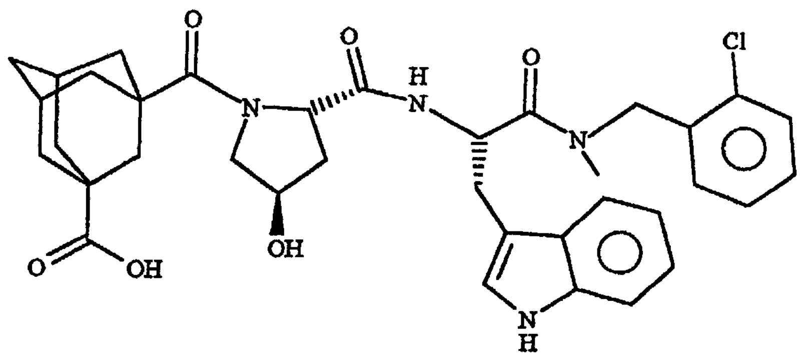

Fp.: 200-204 °C.
$[\alpha]_D^{20}$ = - 7 ° (DMSO).

39.)

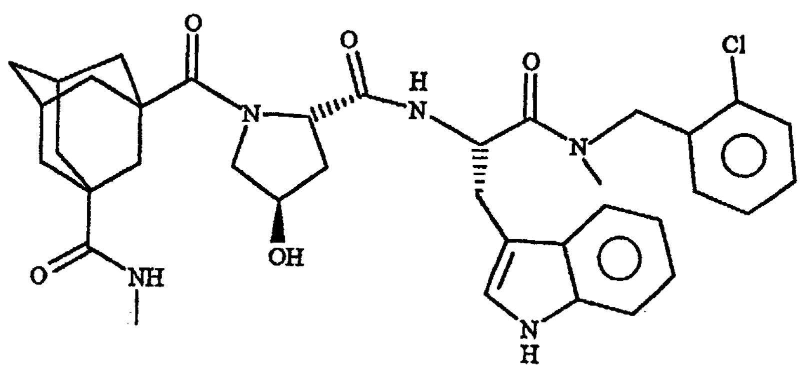

Fp.: 120-125 °C.
$[\alpha]_D^{20}$ = - 5,4 ° (DMSO).

40.)

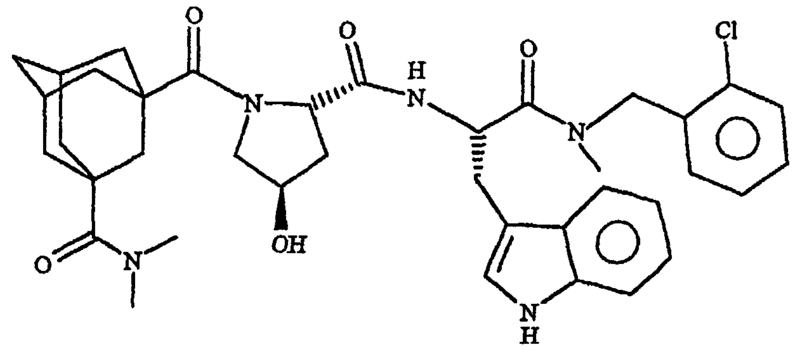

Fp.: 120-125 °C.
$[\alpha]_D^{20}$ = - 5,6 ° (DMSO).

41.)

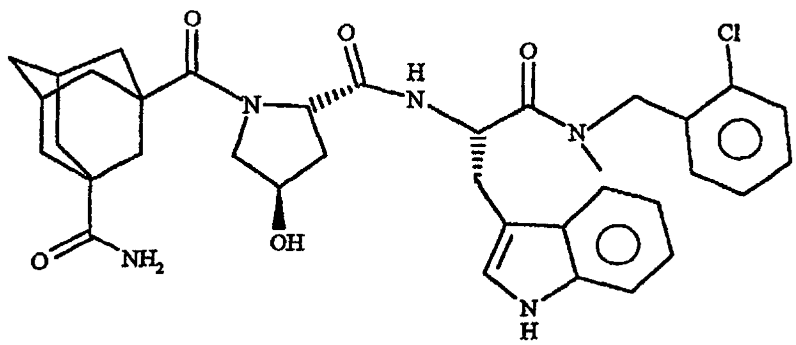

Fp.: 142-147 °C.
$[\alpha]_D^{20}$ = - 1,8 ° (DMSO).

42.)

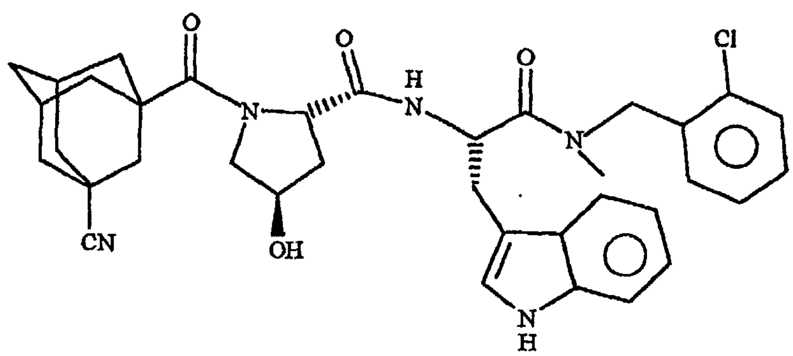

43.)

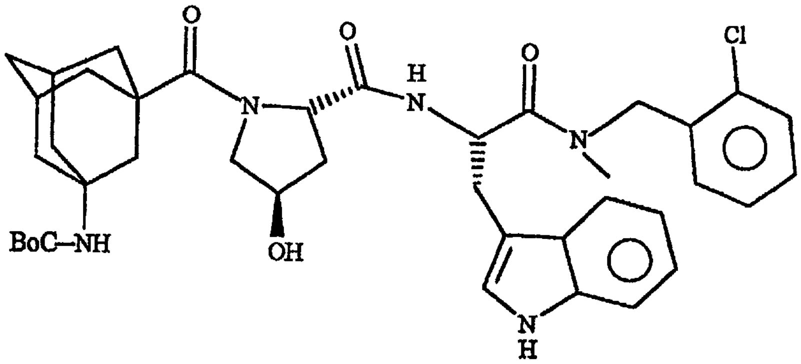

Fp.: 146-148 °C.
$[\alpha]_D^{20}$ [= - 3,8 ° (DMSO).

44.)

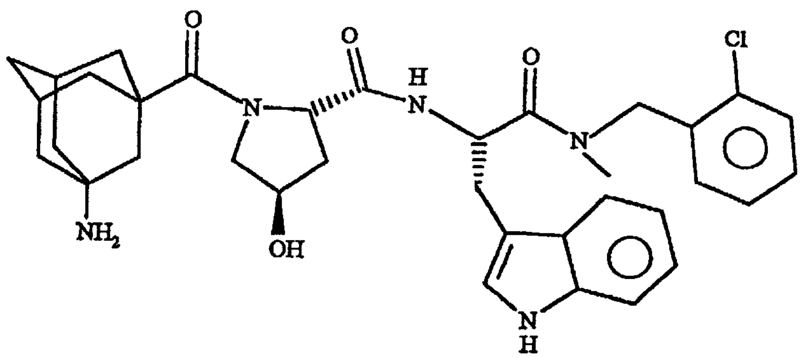

Fp.: 198-202 °C.
$[\alpha]_D^{20}$ = - 7,4 ° (DMSO).

45.)

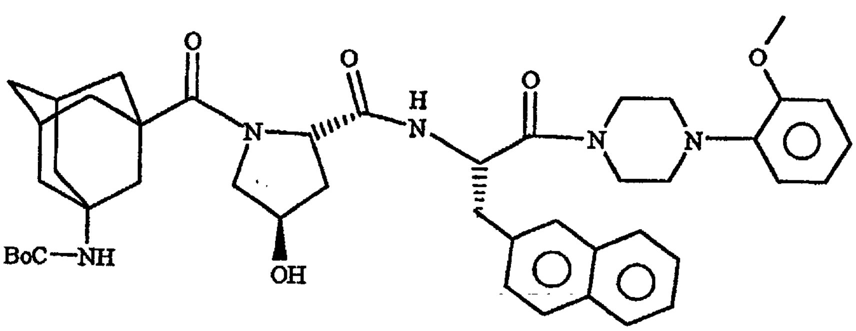

Fp.: 130-138 °C.
$[\alpha]_D^{20}$ = - 30 ° (DMSO).

46.)

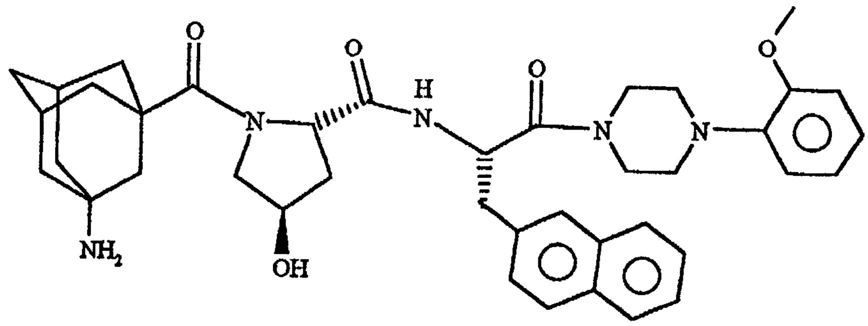

Fp.: 194-197 °C.
$[\alpha]_D^{20}$ = - 30 ° (DMSO).

47.)

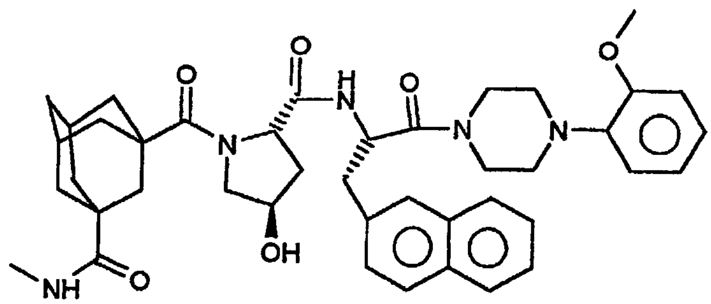

Fp.: 122-127 °C.
$[\alpha]_D^{20}$ = - 35 ° (DMSO)

48.)

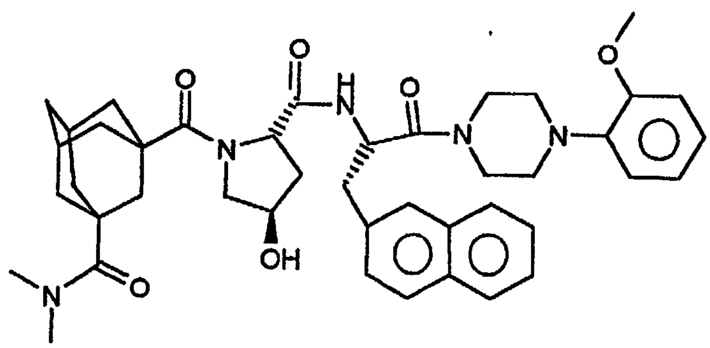

Fp.: 116-122°C;
$[\alpha]_D^{20}$ = - 33,4 ° (DMSO).

49.)

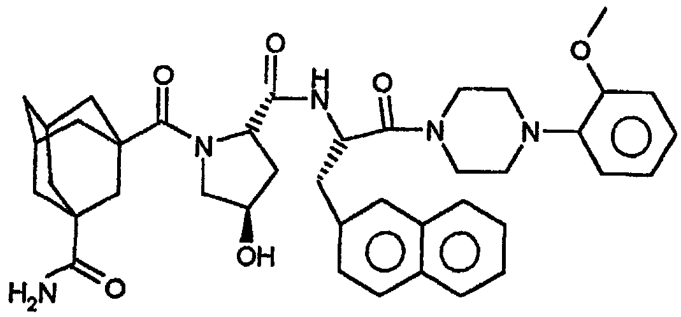

Fp.: 130-138°C.
$[\alpha]_D^{20}$ = - 31,6° (DMSO)

50.)

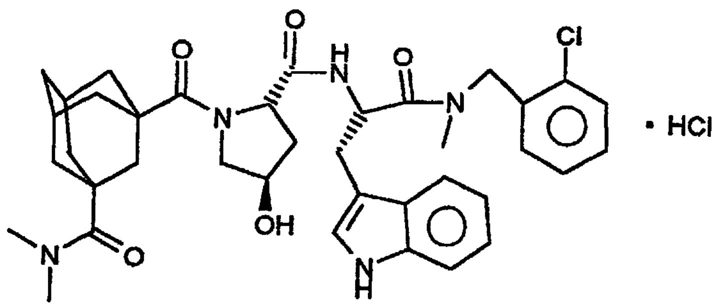

Fp.: 128-138 °C.
$[\alpha]_D^{20}$ = - 6,6 ° (DMSO).

51.)

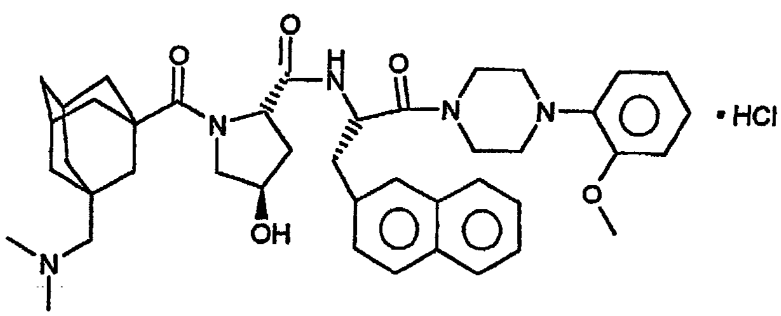

Fp.: 128-138 °C.
$[\alpha]_D^{20}$ = - 30° (DMSO).

52.)

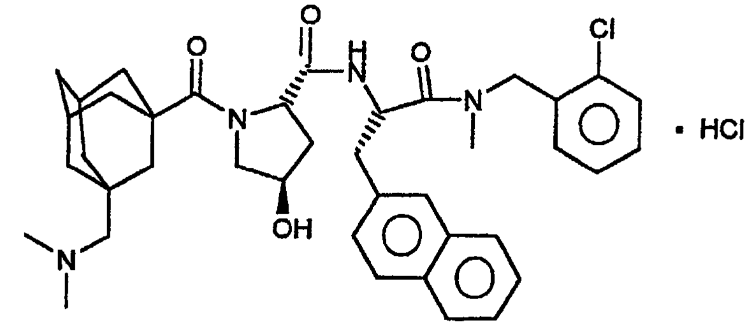

Fp.: 130-135 °C.
$[\alpha]_D^{20}$ = -18 ° (DMSO)

53.)

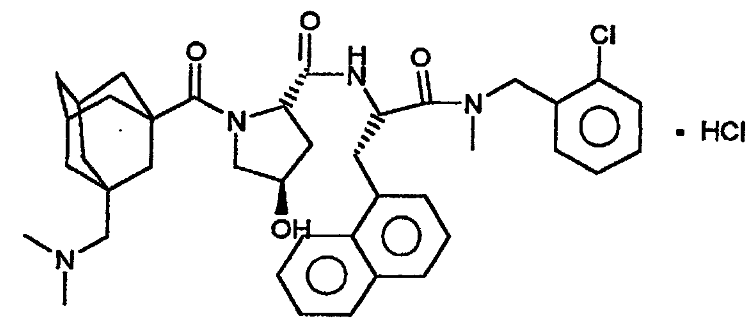

Fp.: 130-135 °C.
$[\alpha]_D^{20}$ = 0° (DMSO).

54.)

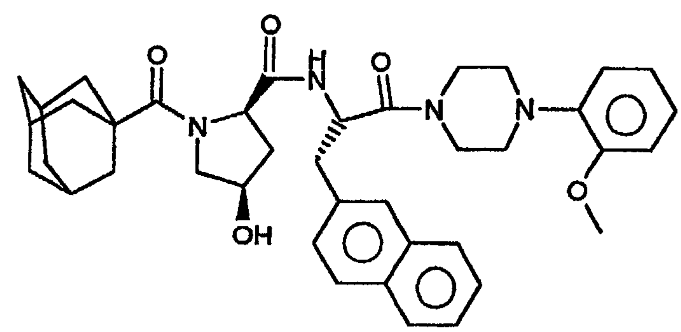

Fp.: 103-106 °C.
$[\alpha]_D^{20}$ = - 45,4 ° (DMSO).

55.)

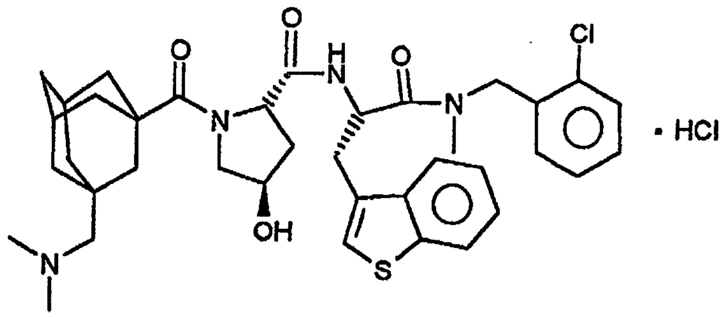

Fp.: 129 - 136 °C.
$[\alpha]_D^{20}$ = - 17,6 °(DMSO).

56.)

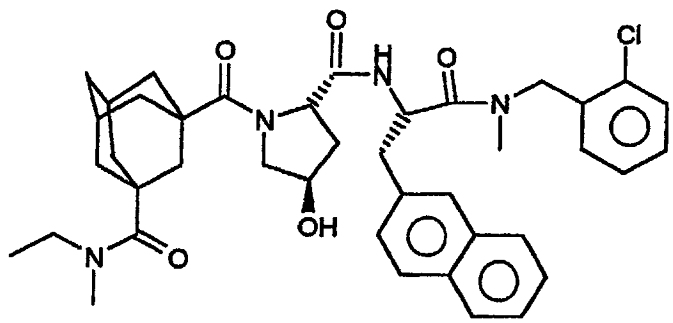

Fp.: 94-98 °C.
$[\alpha]_D^{20}$ = - 19,4 ° (DMSO).

57.)

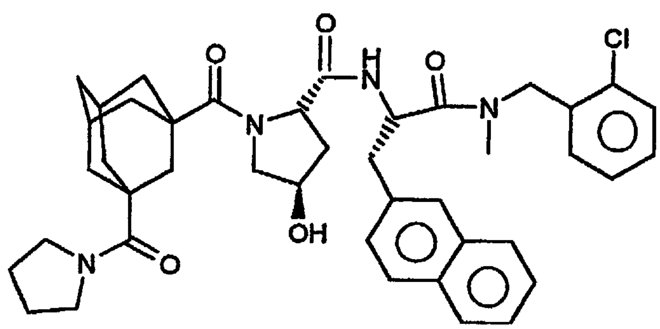

Fp.: 107-110 °
$[\alpha]_D^{20}$ = - 18,4 ° (DMSO).

58.)

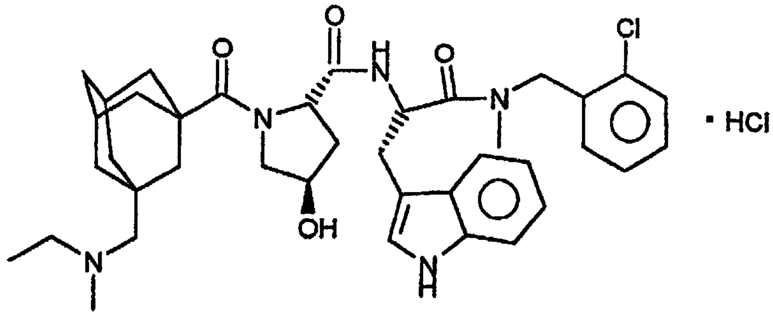

Fp.: 126-128 °C.
$[\alpha]_D^{20}$ = - 8° (DMSO).

59.)

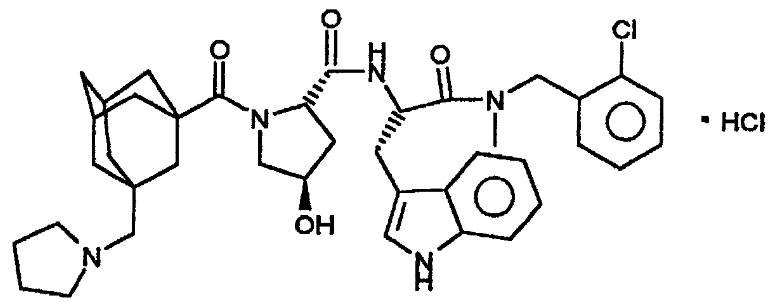

Fp.: 153-158 °C.
$[\alpha]_D^{20}$ = -7,8° (DMSO)

60.)

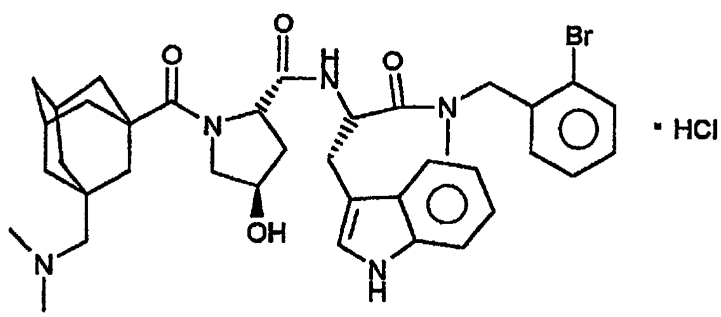

Fp.: 153-163 °C (Zers.)
$[\alpha]_D^{20}$ = - 8,2 ° (DMSO)

61.)

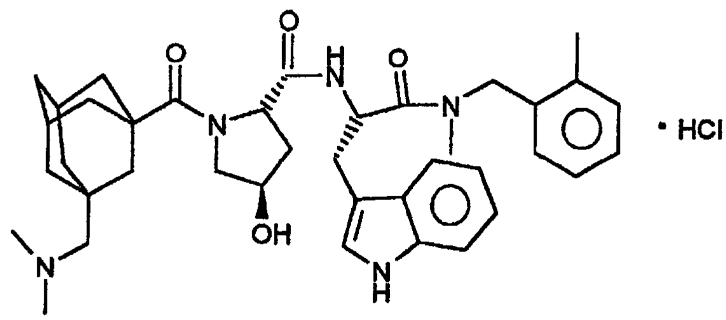

Fp.: 156-164 °C (Zers.)
$[\alpha]_D^{20}$ = - 9,4° (DMSO)

62.)

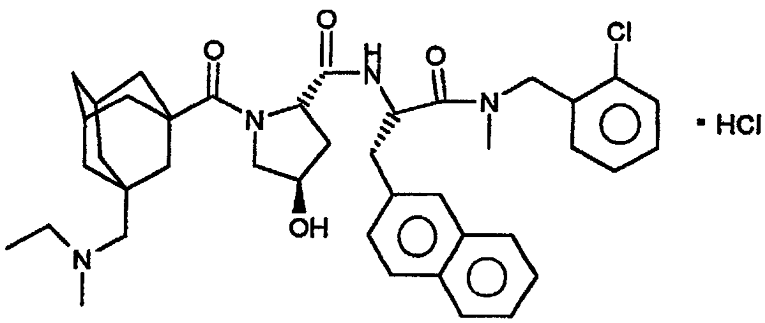

Fp.: 129-136 °C.
$[\alpha]_D^{20}$ = - 19,6 ° (DMSO)

63.)

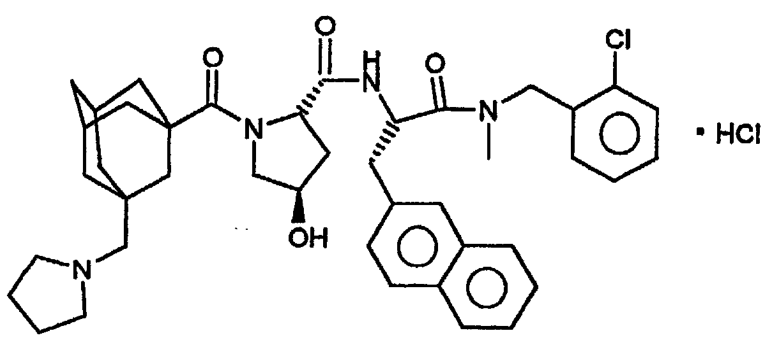

Fp.: 139-146 °C.
$[\alpha]_D^{20}$ = - 18,6° (DMSO)

64.)

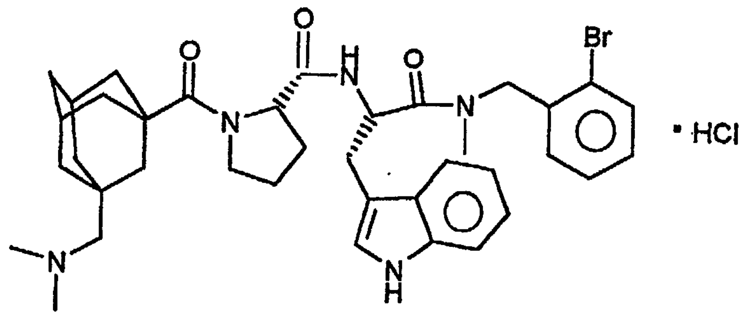

Fp.: 129-135 °C.
$[\alpha]_D^{20}$ [= - 17,4 ° (DMSO)

65.)

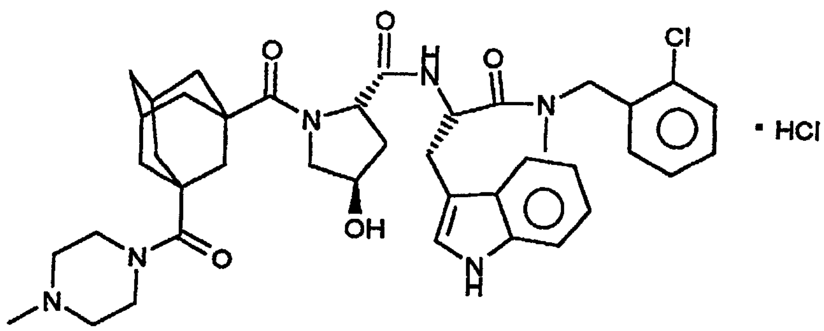

Fp.: 172-176 °C;
$[\alpha]_D^{20}$ = -7,8° (DMSO)

66.)

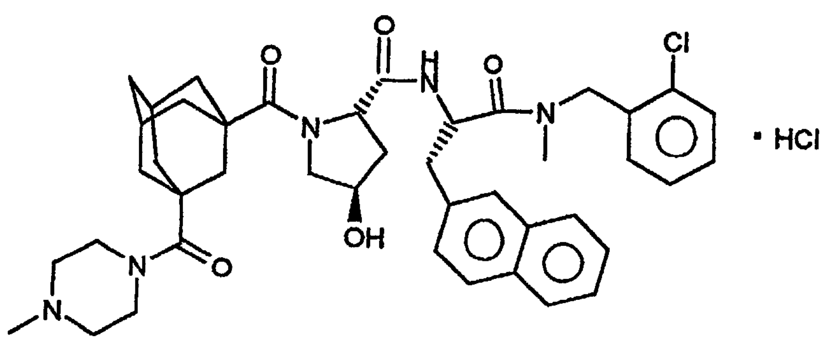

Fp.: 138-145 °C;
$[\alpha]_D^{20}$ = -18,4 ° (DMSO)

67.)

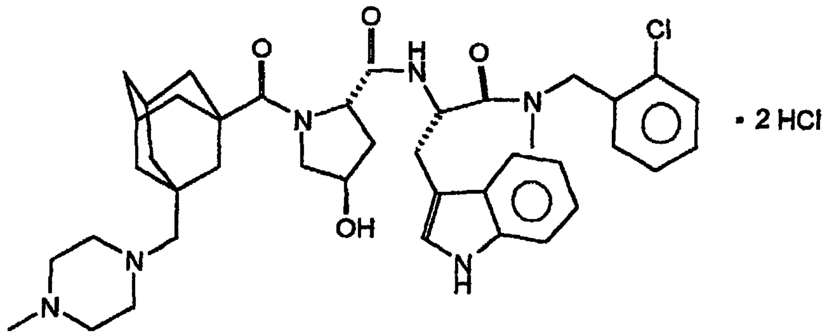

Fp.: 181-187 °C.
$[\alpha]_D^{20}$ = -7,2 (DMSO)

68.)

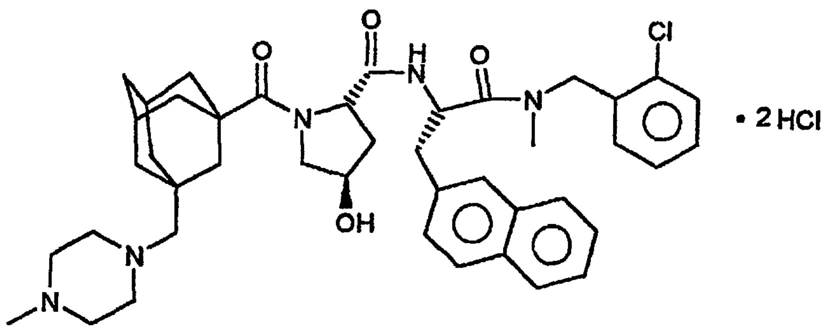

Fp.: 150-155 °C.
$[\alpha]_D^{20}$ = -18° (DMSO)

Von diesen Verbindungen sind Verbindungen 3, 5, 12, 13, 14, 21, 22, 29, 39, 44 und 50 bevorzugt.

In der Darstellung der obigen Formeln wurde die Art verwendet, bei der $CH_3$-Gruppen nicht ausgeschrieben werden.

Verbindung 1 zum Beispiel enthält in der Gruppe $NR^2R^3$ als $R^2$ eine Methylgruppe.

Die erfindungsgemäßen Verbindungen sind wertvolle Neurokinin (Tachykinin)-Antagonisten, die sowohl Substanz P-Antagonismus, als auch Neurokinin A- bzw. Neurokinin-B-antagonistische Eigenschaften besitzen. Sie sind nützlich zur Behandlung von und zur Vorbeugung gegenüber Neurokinin-vermittelten Krankheiten:

Behandlung oder Vorbeugung von entzündlichen und allergischen Erkrankungen der Atemwege, wie Asthma, chronische Bronchitis, hyperreagible Atemwege, Emphysem, Rhinitis, Husten,

der Augen, wie Konjunktivitis und Iritis,

der Haut, wie Dermatitis bei Kontaktekzem, Urtikaria, Psoriasis, Sonnenbrand, Insektenstiche, Juckreiz, sensible oder überempfindliche Haut,

des Magen-Darm-Traktes, wie Magen- und Duodenalgeschwüre, Colitis Ulcerosa, Morbus Crohn, Colon irritabile, M. Hirschsprung,

der Gelenke, wie rheumatoide Arthritis, reaktive Arthritis und Reiter-Syndrom;

zur Behandlung von Erkrankungen des Zentralnervensystems, wie Demenz, M. Alzheimer, Schizophrenie, Psychosen,

Depression, Kopfschmerzen (z.B. Migräne oder Spannungskopfschmerzen), Epilepsie;

Behandlung von Herpes zoster sowie postherpetischer Schmerzen, von Tumoren, Kollagenosen, einer Dysfunktion der ableitenden Hamwege, von Hämorrhoiden, von Übelkeit und Erbrechen, ausgelöst z.B. durch Bestrahlung oder Zytostatikatherapie oder Bewegung und Schmerzzuständen aller Art.

Von besonderem medizinischem Interesse sind Verbindungen deren $NK_1$- und $NK_2$-Werte von ähnlicher Größenordnung sind.

Die Erfindung betrifft daher auch die Verwendung der erfindungsgemäßen Verbindungen als Heilmittel und pharmazeutische Zubereitungen, die diese Verbindungen enthalten. Bevorzugt ist die Anwendung am Menschen. Die Applikation der erfindungsgemäßen Verbindungen kann intravenös, subcutan, intramuskulär, intraperitoneal, intranasal, inhalativ, transdermal, gewünschtenfalls durch lontophorese oder literaturbekannte Enhancer gefördert, und oral erfolgen.

Zur parenteralen Applikation werden die Verbindungen der Formel I oder deren physiologisch veträglichen Salze, eventuell mit den dafür üblichen Substanzen wie Lösungsvermittler, Emulgatoren oder weitere Hilfsstoffe in Lösung, Suspension oder Emulsion gebracht. Als Lösungsmittel kommen z.B. in Frage: Wasser, physiologische Kochsalzlösungen oder Alkohole, z.B. Ethanol, Propandiol oder Glycerin, Zuckerlösungen wie Glucose- oder Mannit-Lösungen oder auch eine Mischung aus verschiedenen Lösungsmitteln.

Außerdem können die Verbindungen durch Implantate, z.B. aus Polylactid, Polyglycolid oder Polyhydroxybuttersäure bzw. intranasale Zubereitungen appliziert werden.

Die erfindungsgemäßen Verbindungen können nach allgemein bekannten Methoden der Amino- und Peptidchemie hergestellt werden, indem man schrittweise die jeweiligen Aminosäuren, Säuren und Amine kondensiert und die so erhaltene Verbindung in freier Form oder in Form des gewünschten Salzes isoliert.

Die erfindungsgemäßen Aminosäurederivate der Formel I

$$R^1\text{-}R^{11}\text{-}A^1\text{-}A^2\text{-}NR^2R^3 \qquad \text{I}$$

können aus den Teilen $R^1$-$R^{11}$OH, H-$A^1$-OH, H-$A^2$-OH und HN($R^3$)$R^2$ aufgebaut werden, wobei die Sequenz der Kupplungen von rechts nach links, von links nach rechts oder durch Kupplung der Einheiten $R^1$-$R^{11}$-$A^1$-OH und H-$A^2$-N($R^3$) $R^2$ (Fragmentkupplungen) erfolgen kann.

Die erfindungsgemäßen Verbindungen können nach allgemein bekannten Methoden der Peptidchemie, wie z.B. in "Houben-Weyl, Methoden der organischen Chemie, Bd. 15/2", beschrieben oder nach der Festphasenpeptidsynthese (z.B. R.C. Sheppard, Int. J. Pept. Prot. Res., 21, 118 [1983]) oder gleichwertigen bekannten Methoden hergestellt werden. Dabei werden die jeweiligen Aminosäuren oder Aminosäureteilsequenzen schrittweise kondensiert und die so erhaltenen Peptide werden in freier Form oder in Form der gewünschten Salze isoliert. Als Aminoschutzgruppen werden die in "Houben-Weyl, Methoden der organischen Chemie, Bd. 15/1" beschriebenen verwendet, wobei in konventionellen Synthesen die Benzyloxycarbonylgruppe (Z) und in Festphasensynthesen die Fluorenylmethoxycarbonylgruppe (Fmoc) bevorzugt wird. Die Seitenkette des Arginins wird im Fall der konventionellen Synthese durch Protonierung geschützt, im Fall der Festphasensynthese wurde die Mtr-Gruppe verwendet. In der Festphasenpeptidsynthese werden auch seitenkettengeschützte Aminosäuren eingesetzt; deren Schutzgruppen z.B. t-Butoxycarbonyl, N($\pi$)-tert. butoxymethyl, Butyl und tert. Butyl sind.
Die speziellen Synthesebedingungen sind dem nachfolgenden Beispiel zu entnehmen.

Zur Synthese der Verbindungen der allgemeinen Formel I nach der Festphasensynthese werden zunächst die Dipeptidcarbonsäuren synthetisiert, die in Lösung zu den Dipeptidamiden umgesetzt werden. Als Ankergruppen sind folgende geeignet

1. Benzylester (G. Barang, R.B. Merrifield, Peptides 2, 1 (1980) Eds. E. Gross, J. Meienhofer, Academic Press, New York)
2. PAM-Anker (R.B. Merrifield, J. Am. Chem. Soc. 85, 2149 (1966))
3. Wang-Anker (S.-S. Wang, J. Am. Chem. Soc. 95, 1328 (1973))
4. SASRIN-Anker (M. Mergler, R. Tanner, J. Gostuli, P. Grogg, Tetrah. Lett. 29, 4005 (1988)).

Beispiel (Verbindung 22)

Syntheseschema

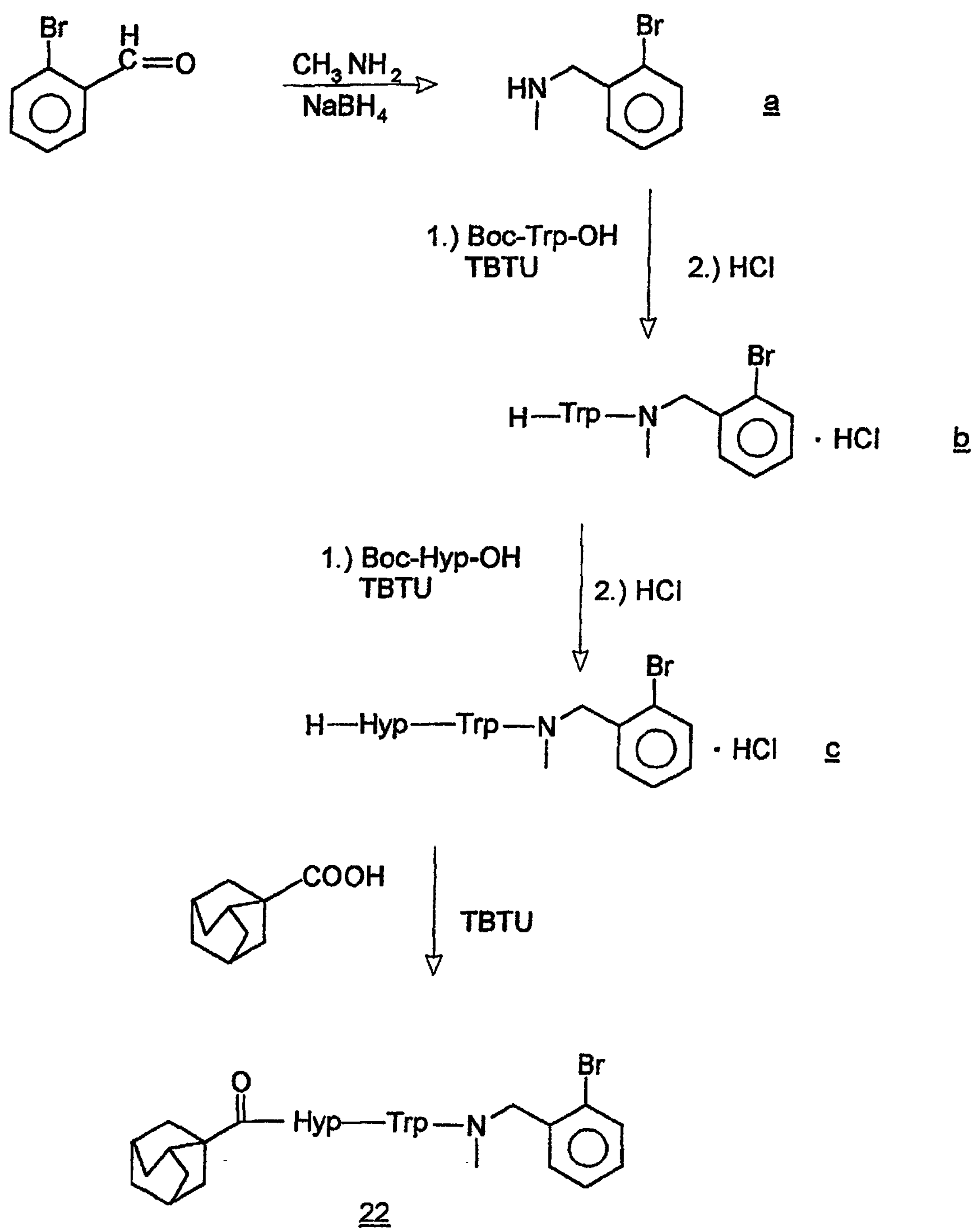


22

Herstellung von a:

29,4 g o-Brombenzaldehyd und 81 ml wässrige, 40%ige Methylamin-lösung werden mit 238 ml THF vereint, und bei RT werden portionsweise innerhalb 25 Min. 19 g $NaBH_4$ hinzugegeben. Man läßt über Nacht bei RT stehen,

engt am Rotationsverdampfer ein, und rührt den Rückstand in Eiswasser ein. Die wässrige Phase wird 2x mit Ether extraliert und die vereinigten Etherphasen unter vermindertem Druck eingeengt. Nach Chromatographie an Kieselgel mit Essigester bzw. Essigester/Methanol (4:1) als Fliessmittel erhält man 18,5 g N-Methyl-2-brombenzylamin (a) in Form einer gelblichen Flüssigkeit. Ausbeute: 58%.

Herstellung von b:

18,4 g Boc-Trp-OH, 12,1 g a und 20,4 g TBTU werden in 430 ml DMF gelöst, mit 17,5 ml TEA versetzt und die Mischung 1 h bei RT gerührt. Man gießt die Reaktionsmischung in 3 l halbkonz. $NaHCO_3$-lsg. und saugt den entstandenen Niederschlag ab. Man löst in ca. 400 ml $CH_2Cl_2$, trennt vom abgeschiedenen Restwasser ab und engt am Rotationsverdampfer ein. Der Rückstand (ca. 28,6 g) wird mit ca 290 ml 4 N HCl in Dioxan und 29 ml Anisol versetzt, duch kurze Behandlung im Ultraschallbad homogenisiert und 45 Min. bei RT stehen gelassen. Es wird unter vermindertem Druck eingeengt, der Rückstand mit Ether verrührt, abgesaugt, mit Ether gewaschen und getrocknet. Man erhält 25,9 g H-Trp-N(Me)-2-brombenzylamid Hydrochlorid (b) in Form beiger Kristalle. Ausbeute: 97%.

Herstellung von c:

12,5 g b, 6,84 g Boc-Hyp-OH, 10,4 g TBTU, 10 ml TEA und 250 ml DMF werden vereinigt und 3 h bei RT gerührt. Die Reaktionsmischung wird in ein Gemisch aus 0,5 l gesättigter $NaHCO_3$-lsg. und 2,2 l Wasser eingerührt, der entstandene Niederschlag abgesaugt, mit Wasser gewaschen und im Exsikkator getrocknet. Die weiße Festsubstanz (15,6 g) wird mit 130 ml 4 N HCl und 13 ml Anisol versetzt, im Ultraschallbad homogenisiert und 75 Min. bei RT stehen gelassen. Es wird am Rotavapor eingeengt, der Rückstand mit Ether verrührt, abgesaugt, mit Ether gewaschen und getrocknet. Man erhält 13,8 g H-Hyp-Trp-N(Me)-2-brombenzylamid Hydrochlorid (c) in Form beiger Kristalle. Ausbeute: 87%.

Herstellung von 22:

0,17 g 3-Noradamantancarbonsäure, 0,54 g c, 0,3 ml TEA, 0,35 g TBTU und 15 ml DMF werden vereinigt, durch Zusatz von weiterem TEA der pH-Wert auf 8-8,5 eingestellt und 135 Min. bei RT belassen. Die Reaktionsmischung wird in 150 ml halbkonz. $NaHCO_3$-lsg. eingerührt und der entstandene Niederschlag abgesaugt, mit Wasser gewaschen und im Exsikkator getrocknet. Die erhaltene Rohsubstanz wird über eine Kieselgelsäule mittels Essigester/Methanol (4:1) chromatographiert. Nach Einengen, Digerieren mit Ether, Absaugen, Waschen mit Ether und Trocknen erhält man 0,28 g 3-Noradamantancarbonyl-Hyp-Trp-N(Me)-2-brombenzylamid (22) in Form beiger Kristalle. Ausbeute 43%.

Fp.: 160 - 165°C; $[\alpha]_D^{20}$ = -7,0° (DMSO)

Pharmazeutische Zubereitunaen:

Injektionslösung

| | | | |
|---|---|---|---|
| 200 mg | Wirksubstanz * | | |
| 1,2 mg | Monokaliumdihydrogenphosphat = $KH_2PO_4$ | | ) |
| 0,2 mg | Dinatriumhydrogenphosphat = $NaH_2PO_4.2H_2O$ | | )(Puffer) ) |
| 94 mg | Natriumchlorid | ) (Isotonans) | |
| oder | | ) | |
| 520 mg | Glucose | ) | |
| 4 mg | Albumin | (Proteasenschutz) | |
| q.s. | Natronlauge | ) | |
| q.s. | Salzsäure | ) ad pH 6 | |
| ad 10 ml | Wasser für Injektionszwecke | | |

Injektionslösung

| | | |
|---|---|---|
| 200 mg | Wirksubstanz* | |
| 94 mg | Natriumchlorid | |
| oder | | |
| 520 mg | Glucose | |
| 4 mg | Albumin | |
| q.s. | Natronlauge | ) |
| q.s. | Salzsäure | ) ad pH 9 |
| ad 10 ml | Wasser für Injektionszwecke | |

| Lyophilisat | |
|---|---|
| 200 mg<br>520 mg<br>4 mg | Wirksubstanz*<br>Mannit (Isotonans/Gerüstbildner)<br>Albumin |
| Lösungsmittel 1 für Lyophilisat | |
| 10 ml | Wasser für Injektionszwecke |
| Lösungsmittel 2 für Lyophilisat | |
| 20 mg | Polysorbat®80 = Tween®80 |
| (oberflächenaktiver Stoff) | |
| 10 ml | Wasser für Injektionszwecke |

*Wirksubstanz: erfindungsgemäße Verbindungen, z.B. die des Beispiels 22.

Dosis für Mensch von 67 kg: 1 bis 500 mg

## Patentansprüche

1. Aminosäurederivat der allgemeinen Formel I

$$R^1\text{-CO-}A^1\text{-}A^2\text{-}NR^2R^3 \qquad (I)$$

oder dessen pharmazeutisch annehmbares Salz, worin
$R^1$ eine der Gruppen

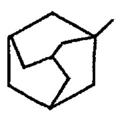
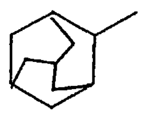
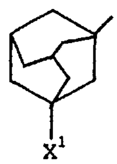
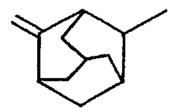
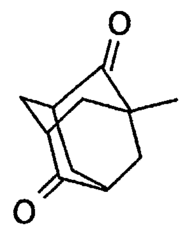

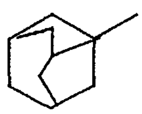
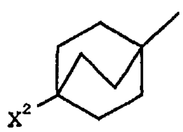
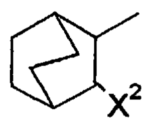

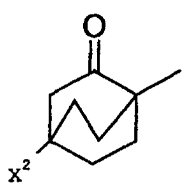
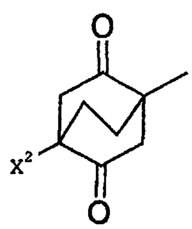
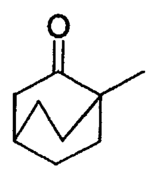
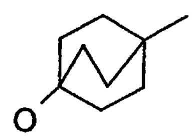

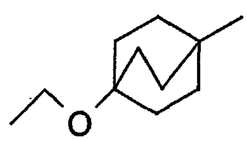
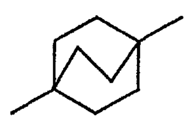

oder

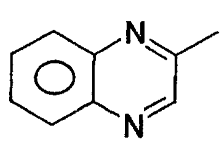

ist, worin

X1 Halogen, COOH, $C(O)NH_2$, C(O)Oalkyl, C(O)NHalkyl, $C(O)N(alkyl)_2$, [worin Alkyl für Methyl, Ethyl, Propyl, Butyl oder Pentyl steht],

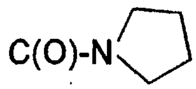 , 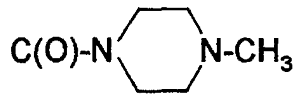 ,

$CH_2NH_2$, $CH_2NH$ alkyl, $CH_2N(alkyl)_2$ [worin Alkyl für Methyl, Ethyl, Propyl, Butyl oder Pentyl steht],

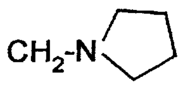 , 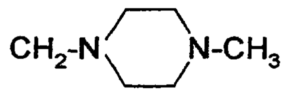 ,

CN, $NH_2$ oder NH(Sch) [worin Sch für Methyloxycarbonyl, Ethyloxycarbonyl oder Phenyl-($C_{1\ oder\ 2}$ alkyl) oxycarbonyl steht, wobei das Phenyl unsubstituiert ist oder durch Halogen, ($C_1$-$C_5$)Alkyl oder ($C_1$-$C_5$)Alkoxy substituiert ist] und

X2 Halogen, Alkyl, OH, O-alkyl, COOH, $C(O)NH_2$, C(O)Oalkyl, C(O)NHalkyl, $C(O)N(alkyl)_2$, [worin Alkyl für Methyl, Ethyl, Propyl, Butyl oder Pentyl steht], CN, $NH_2$ oder NH(Sch) [worin Sch für Methyloxycarbonyl, Ethyloxycarbonyl oder Phenyl - ($C_1$ oder $_2$ alkyl)oxycarbonyl steht, wobei das Phenyl unsubstituiert ist oder durch Halogen, ($C_1$-$C_5$)alkyl oder ($C_1$-$C_5$)alkoxy substituiert ist],

bedeutet.

$A^1$ D- oder L-Serin (Ser), D- oder L-Threonin (Thr), D- oder L-alloThreonin, D-- oder L- Prolin (Pro), D- oder L-Didehydroprolin (Δ Pro), D- oder L-Hydroxyprolin (Pro(OH)), D- oder L-Thiazolidin-4-carbonsäure, D- oder L- Aminoprolin ($Pro(NH_2)$) , D- oder L- Pyroglutaminsäure (pGlu), D- oder L-Hydroxypiperidincarbonsäure, wobei enthaltene Hydroxy- und Aminogruppen durch übliche Schutzgruppen (z.B. Acyl, Carbamoyl oder Aralkyl geschützt sein können;

A2 eine lipophile α-Aminosäure ist, die eine Phenyl-, 1-, 2- oder 3-fach substituierte Phenyl-, Heteroaryl-, oder eine Naphthylgruppe enthält, und diese Ringgruppe durch $-CH_2-$ oder $-CH_2-CH_2-$ vom Backbone der Aminosäure getrennt ist, wobei die Substituenten der Phenylgruppe unabhängig voneinander Halogen, Trihalogenomethyl, Alkoxy oder Alkyl sind;

$R^2$ und $R^3$ unabhängig voneinander Alkyl, Arylalkyl oder Heteroarylalkyl bedeuten (worin Aryl fiir Phenyl, 1, 2 oder 3-fach substituiertes Phenyl oder Naphthyl steht; die Substituenten der Phenylgruppe unabhängig voneinander Halogen, Trihalogenomethyl, Alkoxy, Alkyl, Alkylthio, Hydroxy, Trifluormethoxy, Dialkylamino oder Cyano sind oder 2 benachbarte Positionen der Phenylgruppe durch $-O-(CH_2)_{1\ oder\ 2}-O-$ verbunden sind; Heteroaryl für Indolyl, Pyridyl, Pyrrolyl, Imidazolyl oder Thienyl steht; und die Alkyl- bzw. Alkoxygruppe 1 bis 3 Kohlenstoffatome enthält) oder die Gruppe

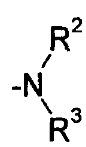

einen Ring der allgemeinen Formel

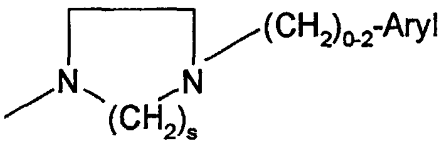

bedeutet, worin s 2 oder 3 ist,
(worin Aryl fiir Phenyl, 1, 2 oder 3-fach substituiertes Phenyl oder Naphthyl steht; die Substituenten der Phenylgruppe unabhängig voneinander Halogen, Trihalogenomethyl, Alkoxy, Alkyl, Cyano, Hydroxy, Nitro, $-CO_2CH_3$, $-CO_2C_2H_5$ oder Alkylthio sind oder 2 benachbarte Positionen der Phenylgruppe durch $-O-(CH_2)_{1-2}-O-$ verbunden sind und Alkyl 1 bis 3 C-Atome enthält).

**2.** Verbindung nach Anspruch 1, worin $A^1$ Prolin, 4-Hydroxyprolin oder Thiazolidin-4-carbonsäure ist.

**3.** Verbindung nach einem der Ansprüche 1 oder 2, worin $A^1$ 4-Hydroxyprolin mit 2-S-Konfiguration ist.

**4.** Verbindung nach einem der Ansprüche 1 bis 3, worin $A^1$

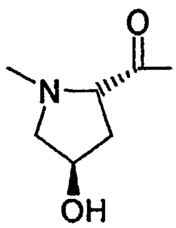

bedeutet.

**5.** Verbindung nach einem der Ansprüche 1 bis 4, worin $A^2$ eine lipophile $\alpha$ -Aminosäure ist, die eine Naphthyl-, Indolyl- oder N-Methylindolyl-Gruppe enthält.

**6.** Verbindung nach Anspruch 5, worin $A^2$

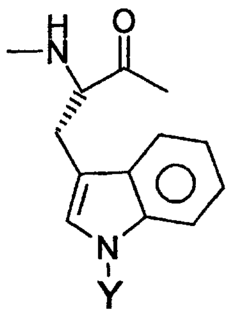

oder

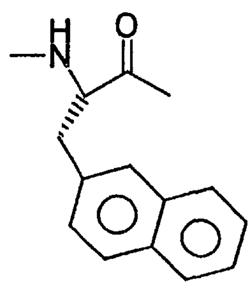

ist, wobei
und Y H oder $CH_3$ bedeutet.

**7.** Verbindung nach einem der Ansprüche 1 bis 6, worin $R^2$ H oder $CH_3$ ist und $R^3$ Benzyl, wobei die darin enthaltene Phenylgruppe durch Methyl, Chlor oder Brom substituiert ist.

**8.** Verbindung nach Anspruch 7, worin $R^3$ 2-Methylbenzyl oder 2-Brombenzyl ist.

9. Verbindung nach einem der Ansprüche 1 bis 6, worin die Gruppe

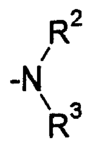

einen Ring

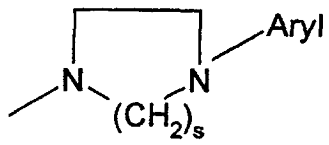

bedeutet, worin s 2 ist .

10. Verbindung nach Anspruch 1, ausgewählt aus der Gruppe bestehend aus

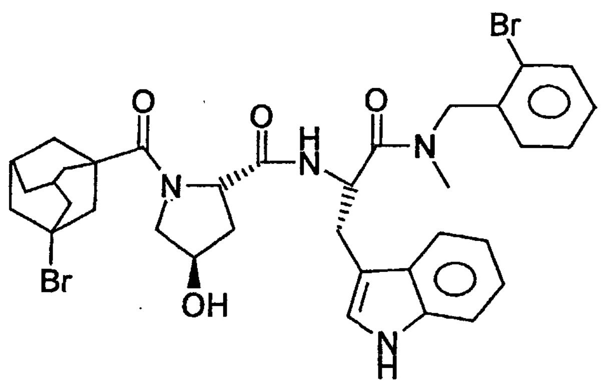

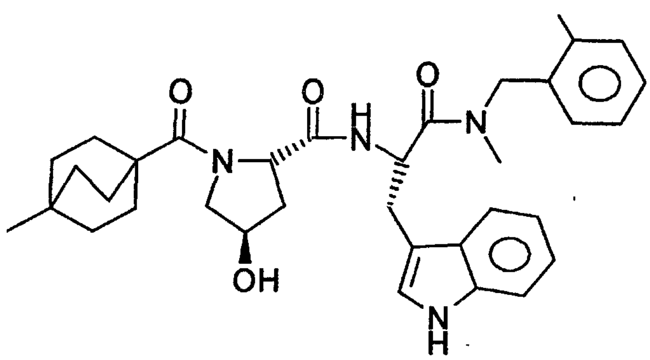

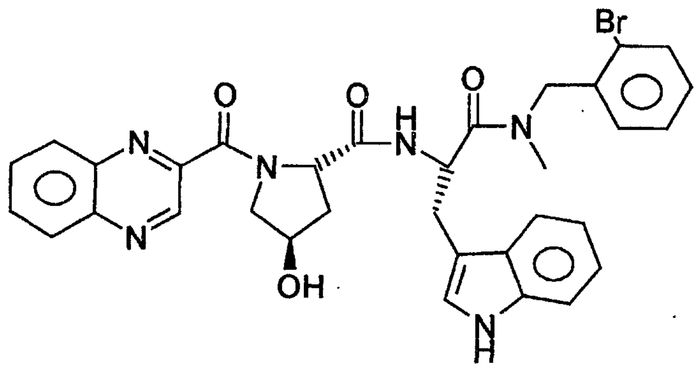

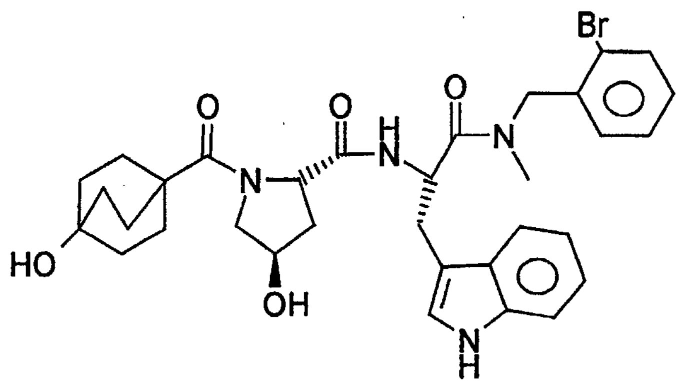

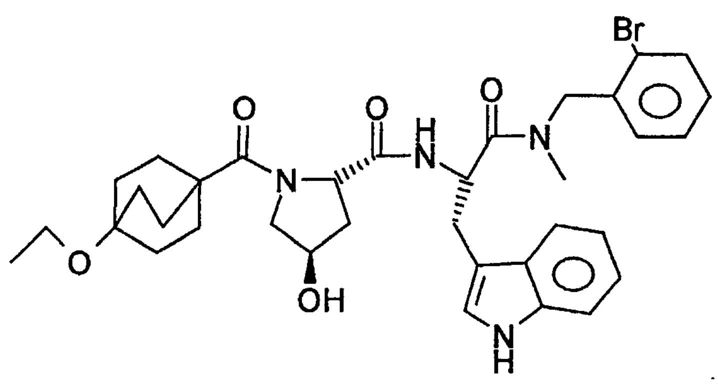

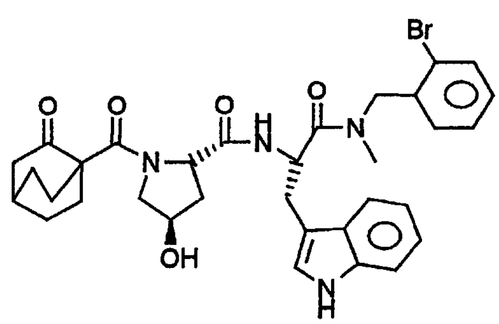

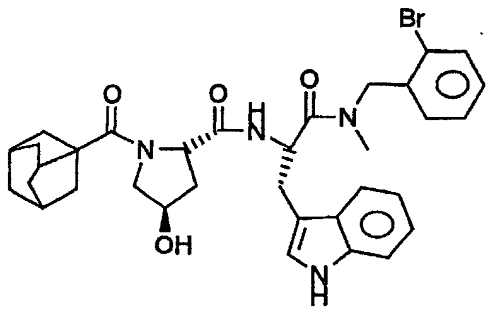

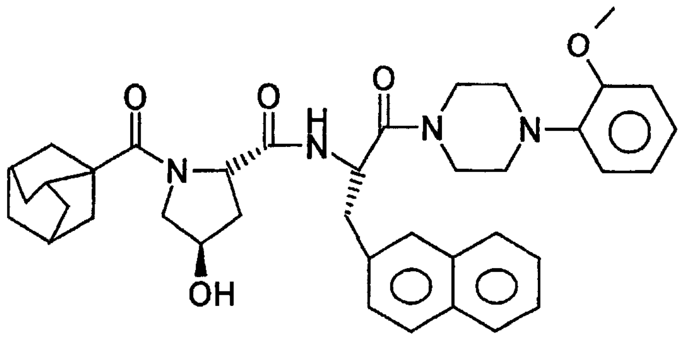

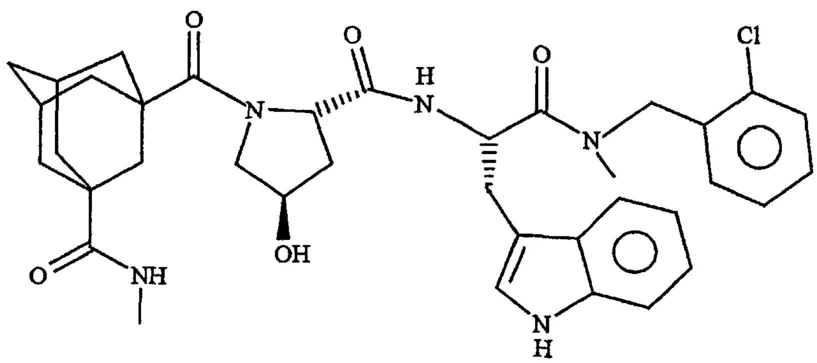

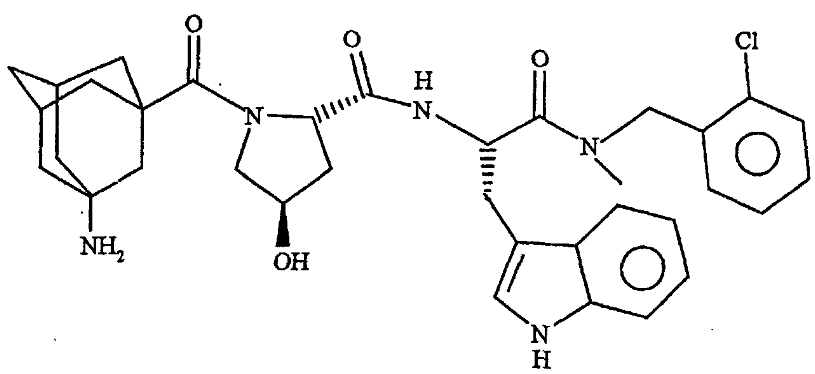

und

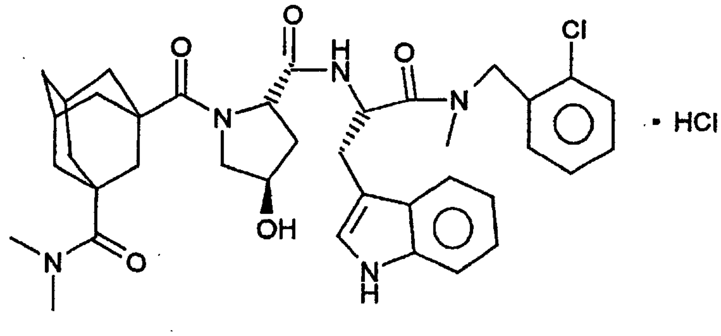

oder deren pharmazeutisch annehmbaren Salze.

**11.** Verfahren zur Herstellung einer Verbindung nach einem der Ansprüche 1 bis 10 oder deren Salze, **dadurch gekennzeichnet, daß** man nach bekannten Methoden schrittweise die jeweiligen Aminosäuren, Säuren und Amine kondensiert und die so erhaltene Verbindung in freier Form oder in Form des gewünschten Salzes isoliert.

**12.** Pharmazeutische Zubereitung enthaltend eine Verbindung nach einem der Ansprüche 1 bis 10.

**13.** Verwendung einer Verbindung nach einem der Ansprüche 1 bis 10 zur Herstellung eines Medikaments zur Therapie von und zur Vorbeugung gegenüber Neurokinin-vermittelten Krankheiten.

## Claims

**1.** Amino acid derivative of general formula I

$$R^1 - CO - A^1 - A^2 - NR^2R^3 \qquad (I)$$

or a pharmaceutically acceptable salt thereof, wherein $R^1$ denotes one of the groups

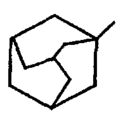
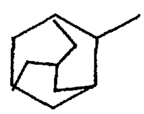
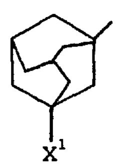
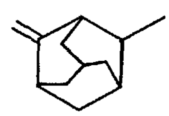

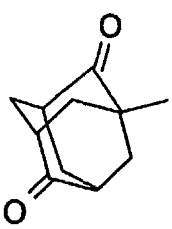
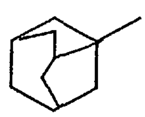
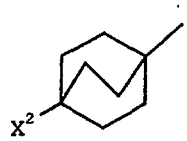
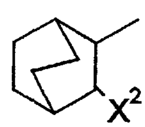

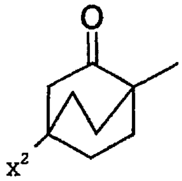 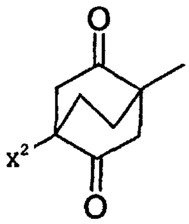 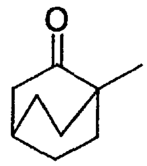 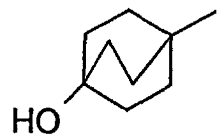

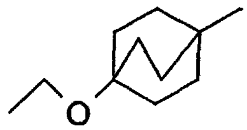 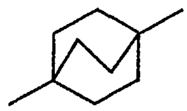 or 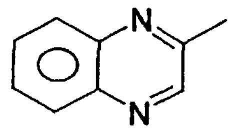 ,

wherein

X1 denotes halogen, COOH, $C(O)NH_2$, C(O)Oalkyl, C(O)NHalkyl, $C(O)N(alkyl)_2$, [wherein alkyl denotes methyl, ethyl, propyl, butyl or pentyl],

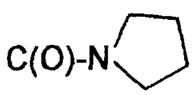 , 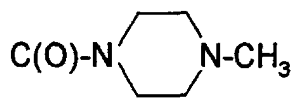 ,

$CH_2NH_2$, $CH_2NHalkyl$, $CH_2N(alkyl)_2$ [wherein alkyl denotes methyl, ethyl, propyl, butyl or pentyl],

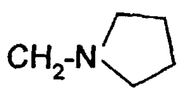 , 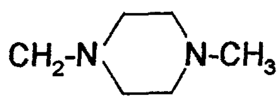 ,

CN, $NH_2$ or NH(Sch) [wherein Sch denotes methyloxycarbonyl, ethyloxycarbonyl or phenyl-($C_{1\ or\ 2}$-alkyl)oxycarbonyl, while the phenyl is unsubstituted or is substituted by halogen, ($C_1$-$C_5$)alkyl or ($C_1$-$C_5$)alkoxy] and

X2 denotes halogen, alkyl, OH, O-alkyl, COOH, $C(O)NH_2$, C(O)Oalkyl, C(O)NHalkyl, $C(O)N(alkyl)_2$, [wherein alkyl denotes methyl, ethyl, propyl, butyl or pentyl], CN, $NH_2$ or NH(Sch) [wherein Sch denotes methyloxycarbonyl, ethyloxycarbonyl or phenyl-($C_{1\ or\ 2}$-alkyl)oxycarbonyl, while the phenyl is unsubstituted or is substituted by halogen, ($C_1$-$C_5$)alkyl or ($C_1$-$C_5$)alkoxy],

$A^1$ is D- or L-serine (Ser), D- or L-threonine (Thr), D- or L-allothreonine, D- or L-proline(Pro), D- or L-didehydroproline (Δ Pro), D- or L-hydroxyproline (Pro(OH)), D- or L-thiazolidine-4-carboxylic acid, D- or L-aminoproline (Pro($NH_2$)) , D- or L-pyroglutamic acid (pGlu), D- or L-hydroxypiperidinecarboxylic acid, while any hydroxy and amino groups present may be protected by conventional protecting groups (e.g. acyl, carbamoyl or aralkyl);

A2 is a lipophilic α-amino acid which contains a phenyl, mono-, di- or trisubstituted phenyl, heteroaryl, or a naphthyl group, and this cyclic group is separated from the backbone of the amino acid by $-CH_2$ or $-CH_2$-$CH_2$-, while the substituents of the phenyl group independently of one another are halogen, trihalomethyl, alkoxy or alkyl;

$R^2$ and $R^3$ independently of one another denote alkyl, arylalkyl or heteroarylalkyl (wherein aryl denotes phenyl, mono-, di- or trisubstituted phenyl or naphthyl; the substituents of the phenyl group independently of one another are halogen, trihalomethyl, alkoxy, alkyl, alkylthio, hydroxy, trifluoromethoxy, dialkylamino or cyano or 2 adjacent positions of the phenyl group are linked by -O-$(CH_2)_{1 \text{ or } 2}$-O-; heteroaryl denotes indolyl, pyridyl, pyrrolyl, imidazolyl or thienyl; and the alkyl or alkoxy group contains 1 to 3 carbon atoms) or the group

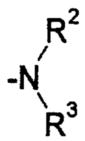

denotes a ring of general formula

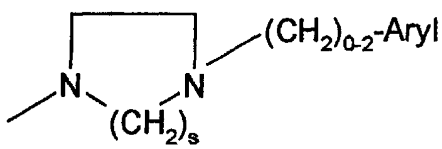

wherein s is 2 or 3,

(wherein aryl denotes phenyl, mono-, di- or trisubstituted phenyl or naphthyl; the substituents of the phenyl group independently of one another denote halogen, trihalomethyl, alkoxy, alkyl, cyano, hydroxy, nitro, $-CO_2CH_3$, $-CO_2C_2H_5$ or alkylthio or 2 adjacent positions of the phenyl group are linked by -O-$(CH_2)_{1-2}$-O- and alkyl contains 1 to 3 carbon atoms).

**2.** Compound according to claim 1, wherein $A^1$ is proline, 4-hydroxyproline or thiazolidine-4-carboxylic acid.

**3.** Compound according to one of claims 1 and 2, wherein $A^1$ is 4-hydroxyproline with a 2-S-configuration.

**4.** Compound according to one of claims 1 to 3, wherein $A^1$ denotes

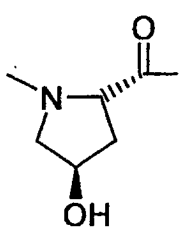

**5.** Compound according to one of claims 1 to 4, wherein $A^2$ is a lipophilic $\alpha$-amino acid which contains a naphthyl, indolyl or N-methylindolyl group.

**6.** Compound according to claim 5, wherein $A^2$ is

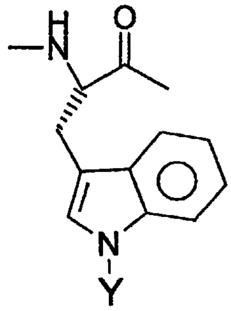

or

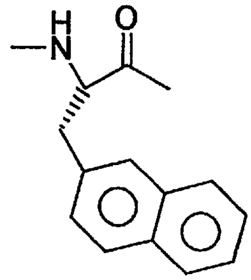

and Y is H or $CH_3$.

**7.** Compound according to one of claims 1 to 6, wherein $R^2$ is H or $CH_3$ and $R^3$ is benzyl, while the phenyl group contained therein is substituted by methyl, chlorine or bromine.

**8.** Compound according to claim 7, wherein $R^3$ is 2-methylbenzyl or 2-bromobenzyl.

**9.** Compound according to one of claims 1 to 6, wherein the group

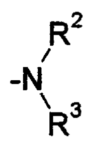

denotes a ring

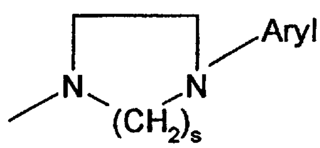

wherein s is 2.

**10.** Compound according to claim 1, selected from among

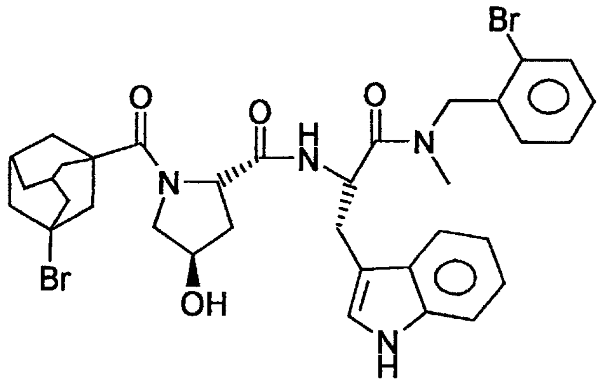

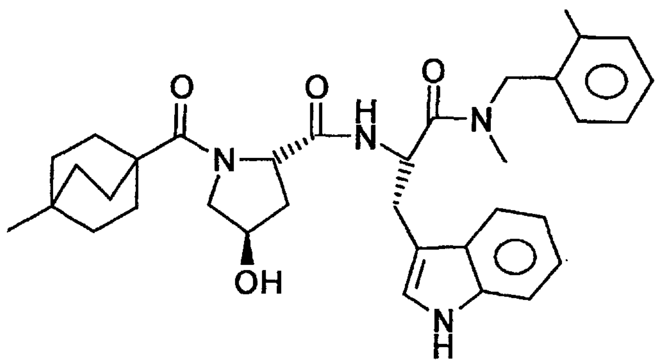

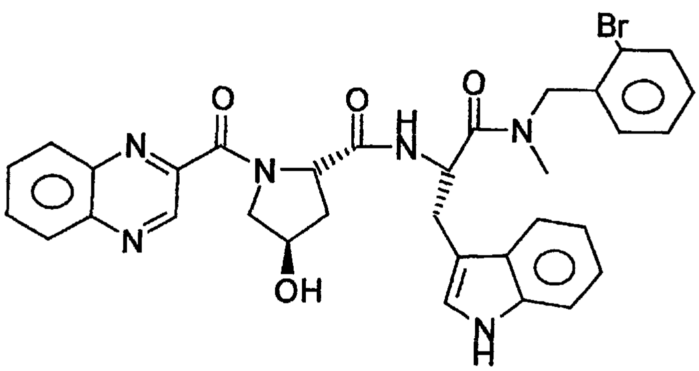

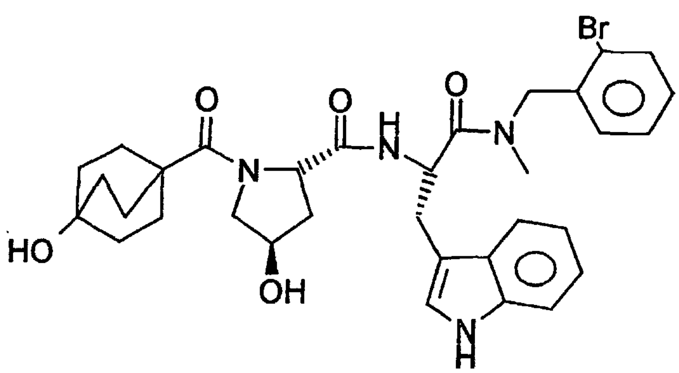

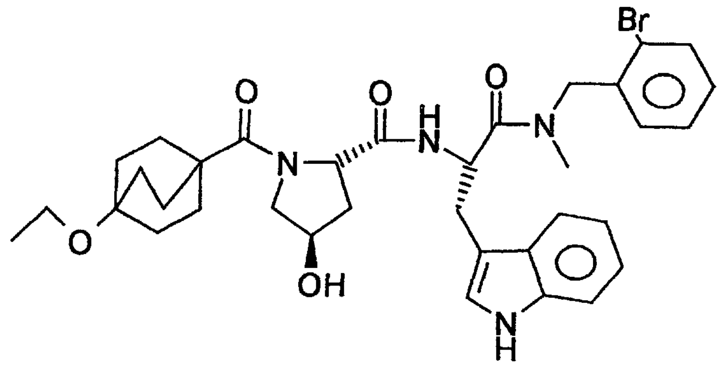

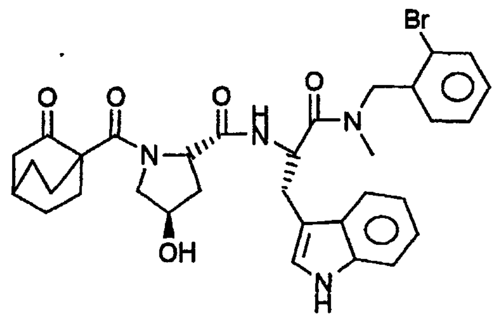

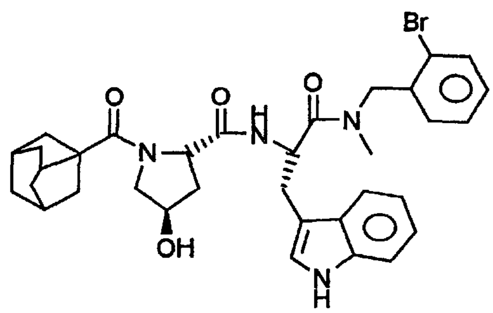

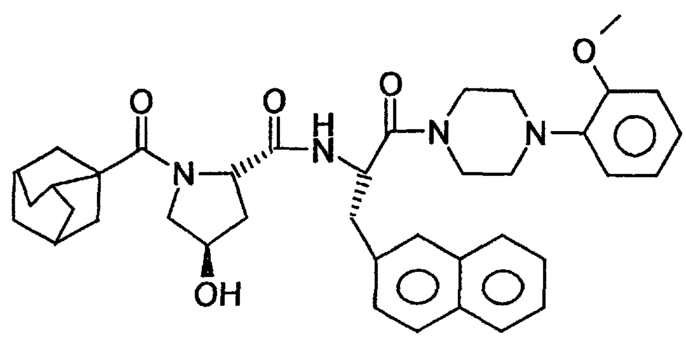

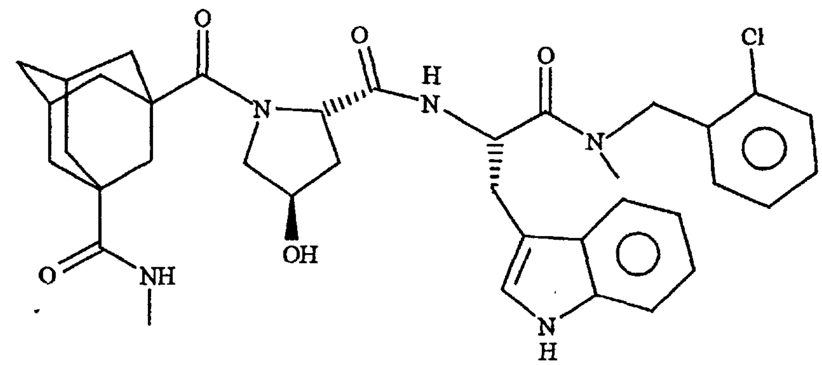

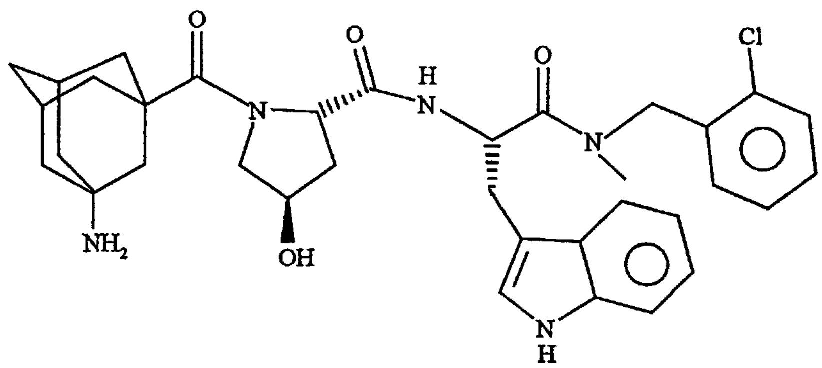

and

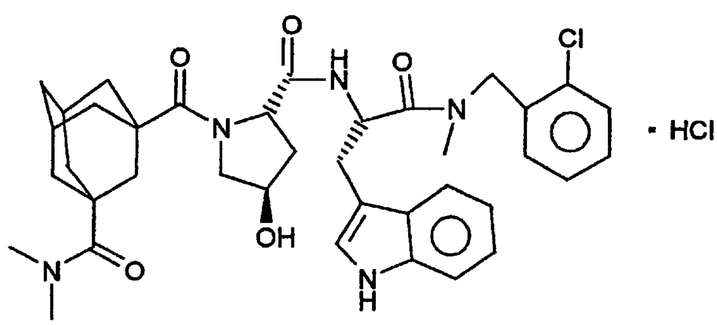

or the pharmaceutically acceptable salts thereof.

11. Process for preparing a compound according to one of claims 1 to 10 or the salts thereof, **characterised in that** the particular amino acids, acids and amines are condensed step-by-step using known methods and the compound thus obtained is isolated in free form or in the form of the desired salt.

12. Pharmaceutical preparation containing a compound according to one of claims 1 to 10.

13. Use of a compound according to one of claims 1 to 10 for preparing a medicament for the treatment and prevention of neurokinin-mediated diseases.

## Revendications

1. Dérivé d'aminoacide de formule générale I

$$R^1\text{-CO-}A^1\text{-}A^2\text{-}NR^2R^3 \qquad (I)$$

ou son sel pharmaceutiquement acceptable, où
$R^1$ représente l'un des groupes

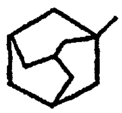
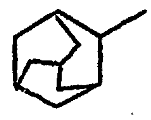
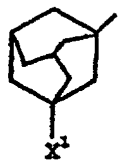
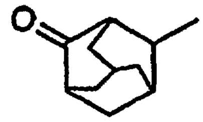
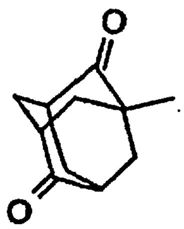
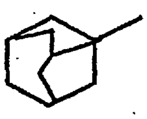
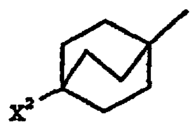
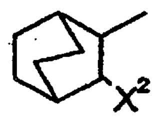
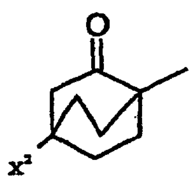
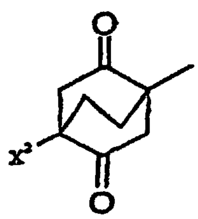
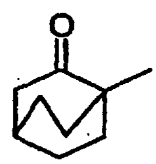
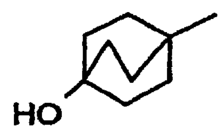
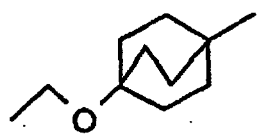
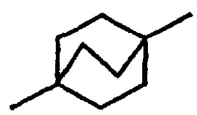

ou

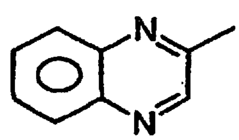

où
$X^1$ représente halogène, COOH, $C(O)NH_2$, C(O)Oalkyle, C(O)NHalkyle, $C(O)N(alkyle)_2$ [où alkyle représente méthyle, éthyle, propyle, butyle ou pentyle],

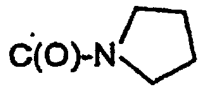, 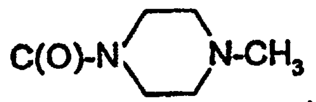,

$CH_2NH_2$, $CH_2NH$alkyle, $CH_2N$(alkyle)$_2$ [où alkyle représente méthyle, éthyle, propyle, butyle ou pentyle],

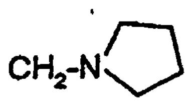, 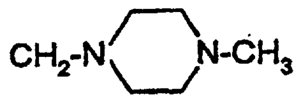,

CN, $NH_2$ ou NH(Sch) [où Sch représente méthyloxycarbonyle, éthyloxycarbonyle ou phényl-(alkyle en $C_{1\ ou\ 2}$) oxycarbonyle, où le phényle est non substitué ou substitué par halogène, alkyle en $C_1$-$C_5$ ou alcoxy en $C_1$-$C_5$] et $X^2$ représente halogène, alkyle, OH, O-alkyle, COOH, C(O)$NH_2$, C(O)Oalkyle, C(O)NHalkyle, C(O)N(alkyle)$_2$ [où alkyle représente méthyle, éthyle, propyle, butyle ou pentyle], CN, $NH_2$ ou NH(Sch) [où Sch représente méthyloxycarbonyle, éthyloxycarbonyle ou phényl-(alkyle en $C_{1\ ou\ 2}$)oxycarbonyle, où le phényle est non substitué ou substitué par halogène, alkyle en $C_1$-$C_5$ ou alcoxy en $C_1$-$C_5$],

$A^1$

représente la D-ou L-sérine (Ser), la D- ou L-thréonine (Thr), la D- ou L-allothréonine, la D- ou L-proline (Pro), la D- ou L-didéshydroproline (Δpro), la D- ou L-hydroxyproline (Pro(OH)), l'acide D- ou L-thiazolidine-4-carboxylique, la D- ou L-aminoproline (Pro($NH_2$)), l'acide D- ou L-pyroglutamique (pGlu), l'acide D- ou L-hydroxypipéridinecarboxylique, où les groupes hydroxyle et amino contenus peuvent être protégés par des groupes protecteurs courants (par exemple acyle, carbamoyle ou aralkyle) ;

A2

est un α-aminoacide lipophile qui contient un groupe phényle, phényle substitué 1, 2 ou 3 fois, hétéroaryle ou un groupe naphtyle, et ce groupe cyclique est séparé du squelette de l'aminoacide par $-CH_2-$ ou $-CH_2-CH_2-$, où les substituants du groupe phényle sont indépendamment les uns des autres halogène, trihalogénométhyle, alcoxy ou alkyle ;

$R^2$ et $R^3$

représentent indépendamment l'un de l'autre alkyle, arylalkyle ou hétéroarylalkyle (où aryle représente phényle, phényle substitué 1, 2 ou 3 fois ou naphtyle ; les substituants du groupe phényle sont indépendamment les uns des autres halogène, trihalogénométhyle, alcoxy, alkyle, alkylthio, hydroxyle, trifluorométhoxy, dialkylamino ou cyano ou 2 positions voisines du groupe phényle sont liées par $-O-(CH_2)_{1\ ou\ 2}-O-$ ; hétéroaryle représente indolyle, pyridyle, pyrrolyle, imidazolyle ou thiényle ; et le groupe alkyle ou alcoxy contient 1 à 3 atomes de carbone) ou le groupe

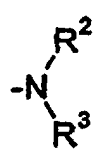

représente un cycle de formule générale

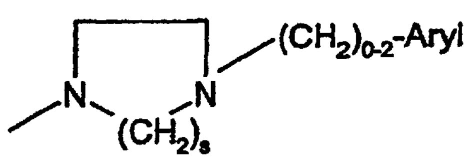

où s est 2 ou 3,

(où Aryl représente phényle, phényle substitué 1, 2 ou 3 fois ou naphtyle ;

les substituants du groupe phényle représentent indépendamment les uns des autres halogène, trihalogénométhyle, alcoxy, alkyle, cyano, hydroxyle, nitro, $-CO_2CH_3$, $-CO_2C_2H_5$ ou alkylthio ou 2 positions voisines du groupe phényle sont liées par $-O-(CH_2)_{1\ ou\ 2}-O-$ et alkyle contient 1 à 3 atomes C).

**2.** Composé selon la revendication 1 où $A^1$ est la proline, la 4-hydroxyproline ou l'acide thiazolidine-4-carboxylique.

**3.** Composé selon l'une des revendications 1 et 2 où $A^1$ est la 4-hydroxyproline de configuration 2-S.

**4.** Composé selon l'une des revendications 1 à 3 où $A^1$ représente

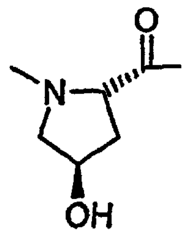

**5.** Composé selon l'une des revendications 1 à 4 où $A^2$ est un $\alpha$-aminoacide lipophile qui contient un groupe naphtyle, indolyle ou N-méthylindolyle.

**6.** Composé selon la revendication 5 où $A^2$ est

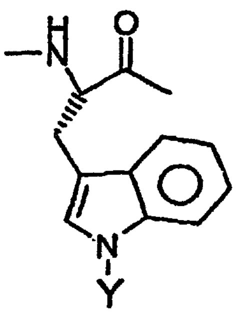

ou

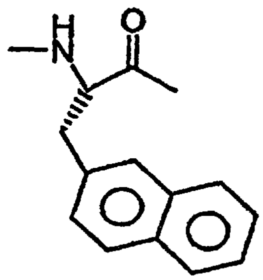

où Y représente H ou $CH_3$.

**7.** Composé selon l'une des revendications 1 à 6 où $R^2$ est H ou $CH_3$ et $R^3$ est benzyle, où le groupe phényle qui y est contenu est substitué par méthyle, le chlore ou le brome.

**8.** Composé selon la revendication 7 où $R^3$ est 2-méthylbenzyle ou 2-bromobenzyle.

**9.** Composé selon l'une des revendications 1 à 6 où le groupe

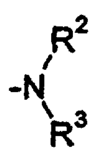

représente un cycle

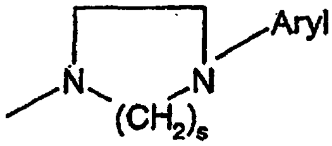

où s est 2.

**10.** Composé selon la revendication 1 choisi dans le groupe consistant en

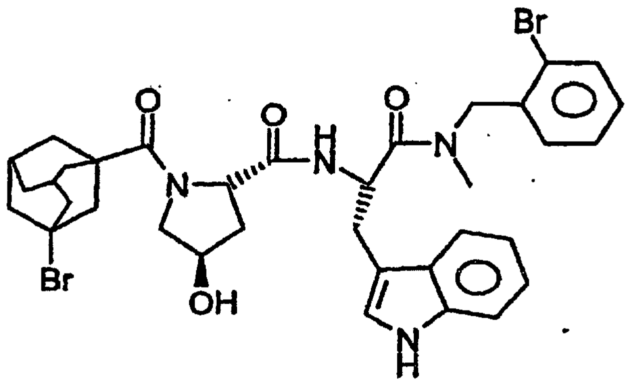

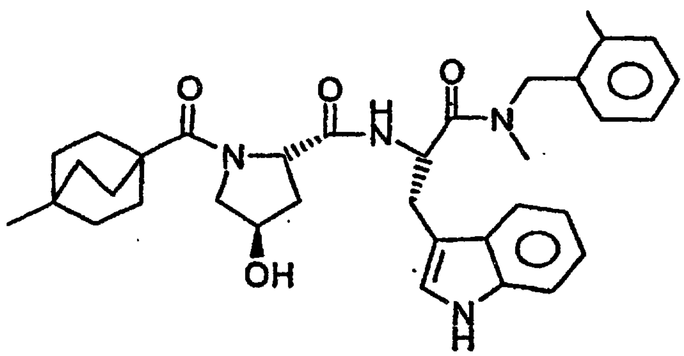

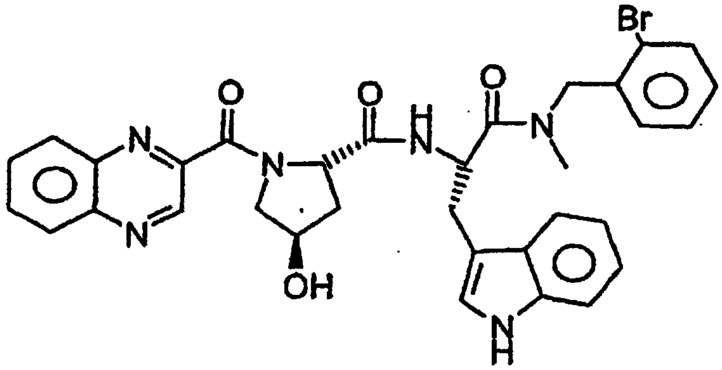

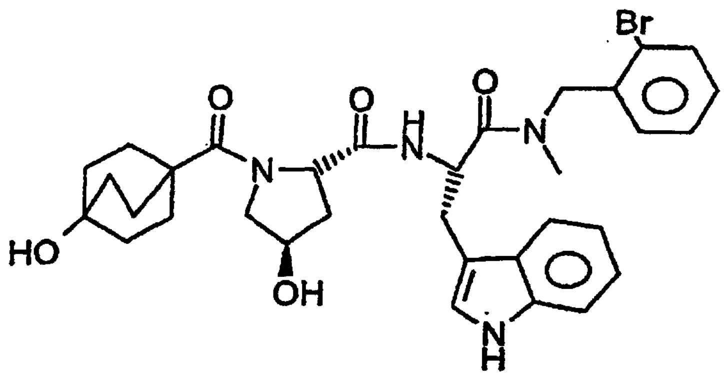

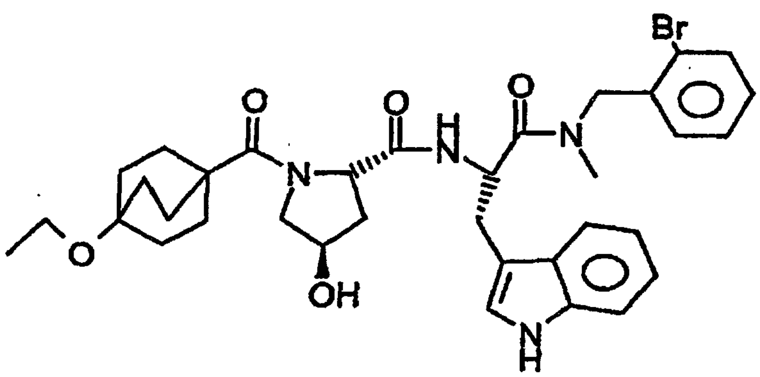

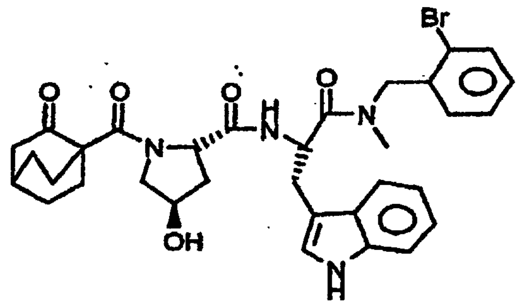

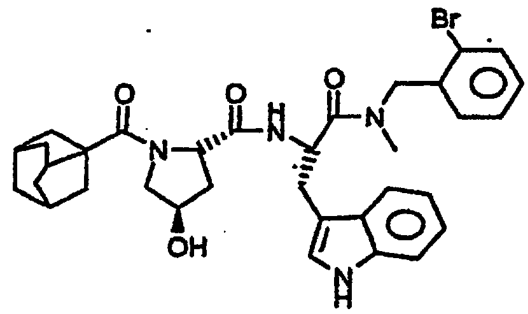

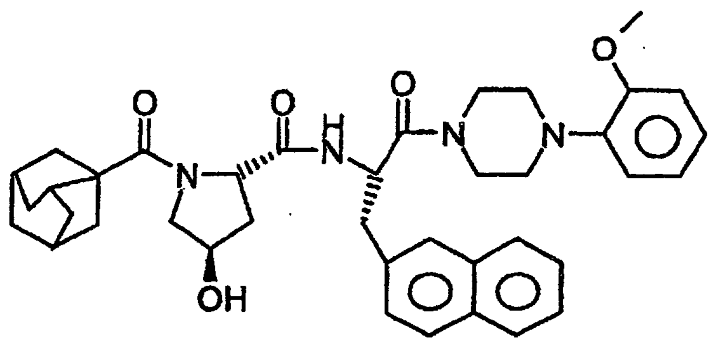

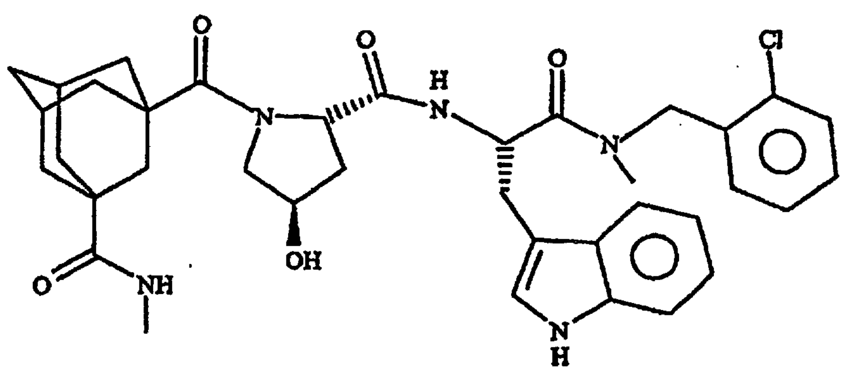

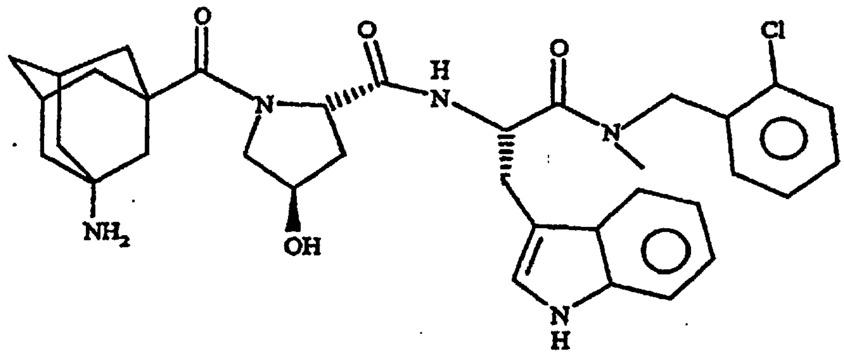

et

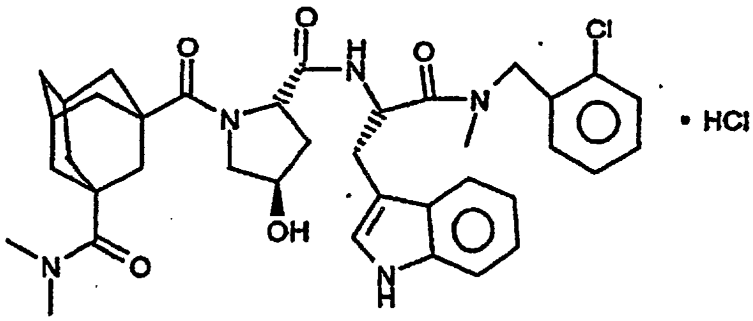

ou ses sels pharmaceutiquement acceptables.

**11.** Procédé de préparation d'un composé selon l'une des revendications 1 à 10 ou de ses sels **caractérisé en ce que** l'on condense par étapes, selon des méthodes connues, les différents aminoacides, acides et amines et on isole le composé ainsi obtenu sous forme libre ou sous forme du sel souhaité.

**12.** Préparation pharmaceutique contenant un composé selon l'une des revendications 1 à 10.

**13.** Utilisation d'un composé selon l'une des revendications 1 à 10 pour la préparation d'un médicament pour la thérapie et pour la prévention de maladies dues à des neurokinines.